# EUROPÄISCHE PATENTANMELDUNG

(11) **EP 4 296 260 A1**
(43) Veröffentlichungstag der Anmeldung: **27.12.2023**
(21) Anmeldenummer: 22180309.1
(22) Anmeldetag: 22.06.2022
(51) Int. Cl.: C07C 263/10, C07C 265/04, C07C 265/14, C07D 307/00, B01J 3/00

(54) **HERSTELLUNG SPEZIELLER ISOCYANATE DURCH CO-PHOSGENIERUNG**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Herstellung von Isocyanaten durch Phosgenierung, insbesondere Gasphasenphosgenierung (GPP) oder Flüssigphasenphosgenierung (FPP), der korrespondierenden Amine, wobei ein Amin-Gemisch aus mindestens einem ersten Amin und mindestens einem von dem ersten Amin verschiedenen zweiten Amin mit Phosgen zu einem Reaktionsgemisch umgesetzt wird, bei dem insbesondere eine höhere Ausbeute und/oder gute Reinheit (in Form einer Erniedrigung des Gehalts an aciden Chlorverbidnungen (AC-Wert) und/oder an hydrolysierbarem Chlor (HC-Wert)) der bei der Herstellung erhaltenen korrespondierenden Isocyanate erzielt werden. Darüber hinaus betrifft die Erfindung ein durch das erfindungsgemäße Verfahren erhältliche oder erhaltene Produkt, vorzugsweise ein durch das erfindungsgemäße Verfahren unmittelbar erhältliche oder erhaltene Produkt sowie die Verwendung dieses Produkts und/oder des Isocyanats- oder Isocyanat-Gemischs, welches durch erfindungsgemäße Verfahren erhalten oder erhältlich ist. Außerdem betrifft die Erfindung eine Phosgenierungsanlage zur Durchführung des erfindungsgemäßen Verfahrens.

## Beschreibung

Die Erfindung betrifft ein Verfahren zur Herstellung von Isocyanaten durch Phosgenierung, insbesondere Gasphasenphosgenierung (GPP) oder Flüssigphasenphosgenierung (FPP), der korrespondierenden Amine, wobei ein Amin-Gemisch aus mindestens einem ersten Amin und mindestens einem von dem ersten Amin verschiedenen zweiten Amin mit Phosgen zu einem Reaktionsgemisch umgesetzt wird, bei dem insbesondere eine höhere Ausbeute und/oder gute Reinheit (in Form einer Erniedrigung des Gehalts an aciden Chlorverbidnungen (AC-Wert) und/oder an hydrolysierbarem Chlor (HC-Wert)) der bei der Herstellung erhaltenen korrespondierenden Isocyanate erzielt werden. Darüber hinaus betrifft die Erfindung ein durch das erfindungsgemäße Verfahren erhältliche oder erhaltene Produkt, vorzugsweise ein durch das erfindungsgemäße Verfahren unmittelbar erhältliche oder erhaltene Produkt sowie die Verwendung dieses Produkts und/oder des Isocyanats- oder Isocyanat-Gemischs, welches durch erfindungsgemäße Verfahren erhalten oder erhältlich ist. Außerdem betrifft die Erfindung eine Phosgenierungsanlage zur Durchführung des erfindungsgemäßen Verfahrens.

Isocyanate, insbesondere Diisocyanate, vereinzelt auch niedermolekulare Triisocyanate, sowie die aus vorstehend genannten Verbindungen zugänglichen höhermolekularen Polyadditionsprodukte mit terminalen Isocyanatgruppen, sind wertvolle Rohstoffe für die Herstellug von Polyurethanen. Die Isocyanatherstellung erfolgt technisch vorteilhaft durch Phosgenierung der ihnen zugrunde liegenden Amine sowohl in kondensierter Phase (Flüssigphosgenierung, im weiteren Text FPP) als auch in der Gasphase (Gasphasenphosgenierung, im weiteren Text GPP). Letztere ist nur bei Aminen durchführbar, die sich unter dem gewählten Druck unzersetzt verdampfen lassen. Ganz allgemein hat sich gezeigt, dass sich viele Isocyanate, die mittels FPP nicht, nur in schlechten Ausbeuten oder mit nicht zufriedenstellender Reinheit herstellbar sind, mittels GPP besser herstellen lassen. Dies trifft insbes. auf Diisocyanate zu, die empfindliche funktionelle Gruppen, wie z.B. Etherbrücken, im Molekülverband tragen (vgl. EP-A 764633 und darin zitierte Quellen).

Auch wenn sich einige Ethergruppen enthaltende Isocyanate mittels GPP in guten Ausbeuten und hoher Reinheit herstellen lassen, trifft das jedoch bei weitem nicht auf alle technisch relevanten Spezies dieser Substanzklasse zu. So ist die Acidität bzw. Gehalt an aciden Chlorverbindungen (im weiteren Text auch: AC, AC-Wert, AC-Gehalt) und der hydrolysierbare Chlorgehalt (im weiteren Text auch: HC, HC-Wert, HC-Gehalt) beispielsweise des 1,5-Diisocyanato-3-oxapentans (im weiteren Text OBDI) nicht hinreichend niedrig, um eine problemlose Weiterverarbeitung dieses Diisocyanates in typischen Polyurethananwendungen zu ermöglichen (s. Vergleichsbeispiele). Weitere Beispiele hierfür sind (poly)cyclische, Ethergruppen aufweisende Amine mit cyclischen Etherbausteinen wie z.B. Zuckerderivate der Furan-, Dihydrofuran-, Tetrahydrofuran-, Sorbitol-, Mannitol- und Iditol-Reihe (Isohexide) wobei es blanglos ist, ob die Aminogruppen direkt oder über weitere Brückenatome an den (Poly)cyclus gebunden sind. Eine destillative Abtrennung der HC-tragenden Verunreinigungen ist bei vielen dieser Verbindungen, insbesondere bei OBDI schwierig, wenn nicht unmöglich.

Die Aufgabe der vorliegende Erfindung bestand demnach darin, ein Verfahren zur Herstellung von Isocyanaten durch Phosgenierung (Gasphasen- und/oder Flüssigphasenphosgnierung) der korrespondierenden Amine sowie eine Phosgenierungsanlage zur Durchführung dieses Verfahrens bereitzustellen. Im Besonderen war es eine weitere Aufgabe der vorliegenden Erfindung, solche hinsichtlich der Phosgenierung "problematischen" Amine (im weiteren Text auch: "Amin(e) 1" bzw. "erste Amin") in technisch gut durchführbarer Weise in die korrespondierenden Isocyanate (im weiteren Text auch: "Isocyanat(e) 1") zu überführen. Dabei sollte insbesondere eine hohe Ausbeute und/oder gute Reinheit in Form einer Erniedrigung des Gehalts an aciden Chlorverbindungen (AC-Wert) und/oder an hydrolysierbaren Chlor (HC-Wert) der bei der Herstellung erhaltenen korrespondierenden Isocyanate erzielt werden.

Gelöst wurde diese Aufgabe durch ein Verfahren zur Herstellung von Isocyanaten durch Phosgenierung der korrespondierenden Amine, wobei ein Amin-Gemisch aus mindestens einem ersten Amin und mindestens einem von dem ersten Amin verschiedenen zweiten Amin mit Phosgen zu einem Reaktionsgemisch umgesetzt wird, dadurch gekennzeichnet, dass
entweder
- das erste Amin mindestens eine Ether-Gruppe enthält; und
- das zweite Amin ausgewählt ist aus der Gruppe, umfassend oder bestehend aus Aminoalkanen mit 5 bis 15 Kohlenwasserstoffatomen, welche keine Ether-Gruppen enthalten;
   oder
- das erste Amin mindestens eine cyclische Ether-Gruppe enthält; und
- das zweite Amin mindestens eine offenkettige Ether-Gruppe und keine cyclischen Ether-Gruppen enthält.

Außerdem betrifft die Erfindung die Verwendung eines Amin-Gemischs aus mindestens einem ersten Amin und mindestens einem von dem ersten Amin unterschiedlichen zweiten Amin in einem Verfahren zur Herstellung der korrespondierenden Isocyanate durch Phosgenierung nach dem erfindungsgemäßen Verfahren zur Erniedrigung des Gehaltes an aciden Chlorverbindungen und/oder des Gehaltes an hydrolysierbarem Chlor der bei der Herstellung erhaltenen korrespondierenden Isocyanate.

Überdies betrifft die Erfindung ein Produkt erhältlich oder erhalten durch das erfindungsgemäßes Verfahren, vorzugsweise unmittelbar erhältlich oder erhalten durch das erfindungsgemäßes Verfahren.

Des Weiteren betrifft die Erfindung die Verwendung des erfindungsgemäßen Produkts und/oder des Isocyanats oder Isocyanat-Gemischs erhalten oder erhältlich nach dem erfindungsgemäßen Verfahren als Komponente zur Herstellung von Polyurethanen, insbesondere Polyurethan-Schaumstoffen, Polyurethan-Beschichtungen und Polyurethan-Klebstoffen, von pharmazeutischen Erzeugnissen, insbesondere Wirkstoffen, sowie von Hilfsstoffen, insbesondere von Hilfsstoffe für die Nassfestausrüstung von Papier und anderen Zelluloseprodukten, Emulgatoren und Verdickungsmitteln.

Die Erfindung betrifft zudem eine Phosgenierungsanlage zur Durchführung des erfindungsgemäßen Verfahrens, umfassend oder bestehend aus
(a) mindestens eine Phosgen-Vorrichtung zur kontinuierlichen Bereitstellung von Phosgen, gegebenenfalls in Kombination mit einem Inertstoff
(b) gegebenenfalls eine Inertstoff-Vorrichtung zur Bereitstellung eines Inertstoffs,
(c) mindestens eine Amin-Vorrichtung zur kontinuierlichen Bereitstellung eines Amin-Gemischs, gegebenenfalls in Kombination mit einem Inertstoff
(d) einen Phosgenierungsreaktor zur Vermischung des Phosgens und des Amin-Gemischs sowie gegebenenfalls des Inertstoffs und Umsetzung des Amin-Gemischs mit dem Phosgen, dadurch gekennzeichnet, dass die Amin-Vorrichtung einen ersten Vorlagebehälter für ein erstes Amin und einen zweiten Vorlagebehälter für ein zweites Amin sowie eine Dosiereinrichtung zur unabhängig voneinander variablen Einstellung der Massenanteile des ersten Amins und des zweiten Amins und optional eine Mischeinrichtung zur Vermischung des ersten Amins und des zweiten Amins zu dem Amin-Gemisch umfasst.

Überraschenderweise wurde gefunden, dass sich Mischungen aus Aminen 1 ("erste Amin") und hinsichtlich der Phosgenierung unproblematischen Amine (im weiteren Text auch: "Amine 2" oder "zweite Amin") weitaus problemloser in die entsprechenden Mischungen aus Isocyanat(en) 1 und Isocyanat(en) 2 überführen lassen, als es bei der alleinigen Phosgenierung der Amine 1 der Fall ist. Dieses erfndungsgemäße Verfahren wird im weiteren Text auch als Co-Phosgenierung, kurz CoPg, bezeichnet. Die hierbei anfallenden Isocyanate 2 sind hinsichtlich Ausbeute und Qualität (insbesondere des Gehalts an aciden Chlorverbindungen (AC-Wert) und/oder an hydrolysierbaren Chlorverbindungen (HC-Wert)) denen mindestens gleichwertig, die bei alleiniger Phosgenierung der Amine 2 resultieren. Besonders überraschend ist es allerdings, dass auch die aus den Aminen 2 zugänglichen Diisocyanate 2 nach dem erfindungsgemäßen Verfahren mitunter in deutlich höherer Reinheit anfallen, als es bei ihrer alleinigen Phosgenierung unter ansonsten vergleichbaren Bedingungen der Fall ist. Näheres dazu ist den Ausführungsbeispielen zu entnehmen. Des Weiteren wurde gefunden, dass Amine mit offenkettigen Ether-Gruppen im Vergleich zu Aminen mit cyclischen Ether-Gruppen relativ gesehen unproblematischer hinsichtlich der Phosgenierung sind. Auch hier hat sich im Rahmen des erfindungsgemäßen Verfahrens gezeigt, dass mit einem Amin-Gemisch aus einem Amin mit mindestens einer cyclischen Ether-Gruppe und einem Amin mit ausschließlich offenkettigen Ether-Gruppen dieselben vorgenannten Verbesserungen hinsichtlich Ausbeute und Qualität (insbesondere des Gehalts an aciden Chlorverbindungen (AC-Wert) und/oder an hydrolysierbaren Chlorverbindungen (HC-Wert)) zu verzeichnen sind. Im Rahmen der Erfindung wird der Gehalt an aciden Chlorverbindungen und der Gehalt an hydrolysierbarem Chlor gemäß der Norm ISO 15028:2014 bestimmt.

Auch wenn die Phosgenierung von Isomerengemischen z.B. der 2,4- sowie 2,6-Diaminotoluole zum techn. etablierten TDI wie auch die Phosgenierung von Isomeren- und Homologengemischen der Amine, die aus der Anilin-Formaldehyd-Kondensation resultieren (MDA --> MDI) wohlbekannte Beispiele für die Phosgenierung von Mischungen verschiedener chemischer Individuen darstellen, ist diese Herangehensweise einzig dem Umstand geschuldet, dass bei der Herstellung der zugrunde liegenden Amine Isomeren- und/oder Homologenmischungen anfallen, die in ihre Einzelbestandteile aufzutrennen weder ökonomisch noch technisch sinnvoll erscheint. Vielmehr bedient man sich hier der Möglichkeit, die mittels Kristallisation (2,4-TDI, sog. "T 100") bzw. Destillation (monomer-MDI, z.B. 4,4'-Bis(isocyanatophenyl)methan, 2,4'-Bis(isocyanatophenyl)methan sowie 2,2'-Bis(isocyanatophenyl)methan und beliebige Mischungen der vorstehend genannten 3 Individuen, im weiteren Text: 2-Kern-MDI)) besser rein zugänglichen Stoffe aus den Isocyanatmischungen zu isolieren als vorher die Aminmischungen entsprechend zu trennen. In der aliphatischen Reihe sind IPDA und das kernhydrierte 2-Kern-MDA (etablierte Akronyme: H12MDA, PACM) Beispiele für zwangsläufig anfallende Mischungen Regio- und/oder Stereoisomere aufweisender Diamine, die nach der Phosgenierung in die entsprechenden Isomerengemische der Diisocyanate IPDI bzw. H₁₂-MDI überführt werden. Bei letzterem ist die Abtrennung des reinen trans-trans-4,4'-Isomers ebenfalls mittels Kristallisation möglich.

Eine bewusst bereitgestellte Mischung zweier (oder mehrerer) chemisch völlig unterschiedlicher und aus völlig unterschiedlichen Herstellprozessen stammender Amine zum Zwecke der Phosgenierung ist bisher lediglich in der DE 2249459 beschrieben worden. Hierbei ist es erfindungswesentlich, ein aromatisches Amin 2 (im Sinne der vorliegenden Anmeldung) als "Basisamin" zur Herstellung aliphatischer Isocyanate aus den entsprechenden Aminen einzusetzen, um für die in der Regel technisch in deutlich geringeren Größenordnungen benötigten aliphatischen Isocyanate keine teure separate Anlage errichten zu müssen. Die Lösung der o.g. technischen Probleme war weder Gegenstand der DE 2249459 noch ist sie nach dieser Lehre lösbar, wie man an den HC-Gehalten der in den Beispielen der DE 2249459 beschriebenen Isocyanate erkennen kann (0,1 % und darüber). Wie das Beispiel 1 der vorliegenden Schrift zeigt, ist das Verfahren der DE 2249459 auch nicht geeignet, die Probleme bei der Herstellung der speziellen Isocyanate im Sinne der vorliegenden Anmeldung zu überwinden.

Auch überrascht es - insbesondere in Anbetracht der mittleren freien Weglänge zwischen den verschiedenen Molekülen in der Gasphase einerseits und in Anbetracht der häufig hohen Verdünnung in der Flüssigphase andererseits - dass die CoPg des ersten und zweiten Amins (Amine 1 und 2) irgendeinen segensreichen Einfluß auf das Gesamtgeschehen hat, abgesehen von den in der DE 2249459 diskutierten, auf der Hand liegenden ökonomischen Vorteilen, da man keine zweite Anlage für die Herstellung spezieller Isocyanate mit geringerer Tonnage, als sie beispielsweise für die MDI-Herstellung üblich ist, benötigt.

Die Erfindung betrifft ein Verfahren zur Herstellung von Isocyanaten durch Phosgenierung der korrespondierenden Amine, wobei ein Amin-Gemisch aus mindestens einem ersten Amin und mindestens einem von dem ersten Amin verschiedenen zweiten Amin mit Phosgen zu einem Reaktionsgemisch umgesetzt wird, dadurch gekennzeichnet, dass
entweder
   - das erste Amin mindestens eine Ether-Gruppe enthält; und
   - das zweite Amin ausgewählt ist aus der Gruppe, umfassend oder bestehend aus Aminoalkanen mit 5 bis 15 Kohlenwasserstoffatomen, welche keine Ether-Gruppen enthalten;
oder
   - das erste Amin mindestens eine cyclische Ether-Gruppe enthält; und
   - das zweite Amin mindestens eine offenkettige Ether-Gruppe und keine cyclischen Ether-Gruppen enthält.

Die Phosgenierung ist vorzugsweise eine Gasphasenphosgenierung (GPP) oder Flüssigphasenphosgenierung (FPP). Das Mischungsverhältnis des ersten und zweiten Amins (Amine 1 und 2) kann im erfindungsgemäßen Verfahren in weiten Grenzen variieren (20 : 1 bis 1 : 20). Vorzugsweise beträgt das Mischungsverhältnis von dem ersten Amin zu dem zweiten Amin 1 : 9 bis 9 : 1, weiter bevorzugt 2 : 8 bis 8 : 2. Auch wenn hohe Anteile an Amin 1 im Amingemisch naheliegenderweise zu einer höheren Ausbeute an dem daraus resultierenden Isocyanat 1 führen, kann es zur Herstellung besonders reiner Isocyanate 1 auch bevorzugt sein, Amin 1 in nicht zu hohem Anteil in Abmischung mit Amin 2 der CoPg zu unterwerfen. Auch kann bei der Gasphasenphosgenierung das Verhältnis von Amin 1 zu Amin 2 (ersten zu zweiten Amin) im Verlaufe der Reaktion verändert werden, bis hin zur ausschließlichen Dosierung des Amines 2 in der Anfahrphase und schließlich der ausschließlichen Dosierung des Amines 1 nach Erreichen eines stabilen Betriebszustandes ("steady state"). Bei dieser speziellen Ausführungsform des Verfahrens macht man sich den Umstand zu Nutze, dass gerade beim Anfahren der Gasphasenphosgenierung häufig kritische Phasen durchlaufen werden, die besser mit dem Amin 2 gemeistert werden, als mit Amin 1 und bei ausschließlicher Dosierung des letzteren in der Anfahrphase zum Abbruch des Versuches führen würden.

Bei der Auswahl an Amin 1 und 2 kann darauf geachtet werden, dass die resultierenden Isocyanate 1 und 2 - beispielsweise mittels Destillation, Extraktion oder Kristallisation - gut voneinander abtrennbar sind. Es ist aber auch möglich, die Mischungen der Isocyanate 1 und 2 - üblicherweise nach destillativer Abtrennung farbgebender und schwersiedender Verunreinigungen - so als Gemisch weiter zu verwenden, wie sie im erfindungsgemäßen Verfahren anfallen.

Diese Herangehensweise ist in der DE 2249459 expressis verbis ausgeschlosssen, da man dort als erfindungswesentlich herausstellt, dass die (beiden) Isocyanate am Ende des Verfahrens getrennt werden können sollen (vgl. DE 2249459, S. 4 letzter Absatz und Ansprüche 1-3).

Insbesondere ist das mindestens eine Ether-Gruppe enthaltende erste Amin, das mindestens eine cyclische Ether-Gruppe enthaltende erste Amin, das Aminoalkane mit 5 bis 15 Kohlenwasserstoffatome umfassende oder daraus bestehende zweite Amin und/oder das mindestens eine offenkettige Ether-Gruppe und keine cyclischen Ether-Gruppen enthaltene zweite Amin ein Diamin.

Das mindestens eine Ether-Gruppe enthaltende erste Amine weist vorzugsweise eine Struktur der allgemeinen Formel X-(-R¹-NHₘ)ₙ auf, wobei
- X für H, NHₘ oder C(R²)ₚ;
- R¹ für, gegebenenfalls substituierte und/oder Heteroatom-aufweisende, aliphatische, (cyclo)aliphatische oder aromatische Reste mit vorzugsweise bis zu 10 Kohlenstoffatomen, wobei R¹ mindestens eine Ethergruppe enthält;
- R² für H, gegebenenfalls substituierte und/oder Heteroatom-aufweisende, aliphatische, (cyclo)aliphatische oder aromatische Reste mit vorzugsweise bis zu 10 Kohlenstoffatomen;
- m für 1 oder 2;
- n für 1, 2 oder 3; und
- p für 1, 2 oder 3
steht.

In einer bevorzugten Ausführungsform ist das mindestens eine Ether-Gruppe enthaltende erste Amin ausgewählt aus der Gruppe, umfassend oder bestehend aus (Tetrahydrofuran-2,5-diyl)dimethanamin (TEFUDA), 3,6-diamino-hexahydrofuro[3,2-b]furan (z.B. IDDA und/oder ISODA), 3,6-Bis(aminomethyl)-hexahydrofuro [3,2-b] furan, Furan-2,5 -diyldimethanamin, (Methan-2,2-diylbis(tetrahydrofuran-5,2-diyl))dimethanamin, (Methan-2,2-diylbis(furan-5,2-diyl))dimethanamin, (Propan-2,2-diylbis(tetrahydrofuran-5,2-diyl))dimethanamin, (Propan-2,2-diylbis(furan-5,2-diyl))dimethanamin; Di(aminoethyl)ether, insbesondere 2,2'-oxybis(ethan-1-amin), 1,1'-oxybis(propan-2-amin), 2-(2-aminoethoxy)propan-1-amin, 2,2'-oxybis(propan-1-amin) und 2-(2-aminopropoxy)propan-1-amin; Di(aminopropyl)ether oder Mischungen davon. Das mindestens eine cyclische Ether-Gruppe enthaltende erste Amin ist vorzugsweise ausgewählt aus der Gruppe, umfassend oder bestehend aus (Tetrahydrofuran-2,5-diyl)dimethanamin (TEFUDA), 3,6-diamino-hexahydrofuro[3,2-b]furan (z.B. IDDA und/oder ISODA), 3,6-Bis(aminomethyl)-hexahydrofuro[3,2-b]furan, Furan-2,5-diyldimethanamin, (Methan-2,2-diylbis(tetrahydrofuran-5,2-diyl))dimethanamin, (Methan-2,2-diylbis(furan-5,2-diyl))dimethanamin, (Propan-2,2-diylbis(tetrahydrofuran-5,2-diyl))dimethanamin, (Propan-2,2-diylbis(furan-5,2-diyl))dimethanamin oder Mischungen davon.

Es ist ferner bevorzugt, wenn das Aminoalkan mit 5 bis 15 Kohlenwasserstoffen umfassende oder daraus bestehende zweite Amin ausgewählt ist aus der Gruppe, umfassend oder bestehend aus 1,5-Diaminopentan; 1,6-Diaminohexan; 1-Amino-3,5,5-trimethyl-5-aminomethylcyclohexan; Amino-[(aminocyclohexyl)methyl]cyclohexan, insbesondere 4,4'-Methylenbis(cyclohexan-1-amin), 2-((4-Aminocyclohexyl)methyl)cyclohexan-1-amin und 2,2'-Methylenbis(cyclohexan-1-amin); 1,3-Bis(aminomethyl)cyclohexan; 1,4-Bis(aminomethyl)cyclohexan; Diaminocyclohexan, insbesondere Cyclohexan-1,2-diamin, Cyclohexan-1,3-diamin und Cyclohexan-1,4-diamin; Methyl-diamino-cyclohexan, insbesondere 4-Methylcyclohexan-1,3-diamin und 2-Methylcyclohexan-1,3-diamin; 4,4'-methylenbis(2-methylcyclohexan-1-amin); oder Mischungen davon. Allerdings können eine Reihe anderer Amine ebenfalls als "Amine 2" geeignet seien, was durch einfache Vorversuche leicht zu ermitteln ist.

Das mindestens eine offenkettige Ether-Gruppen und keine cyclischen Ether-Gruppen enthaltende zweite Amin ist vorzugsweise ausgewählt aus der Gruppe, umfassend oder bestehend aus Di(aminoethyl)ether, Di(aminopropyl)ether, 1,8-Diamino-1,5,8-trimethyl-3,6-dioxaotan, 1,11-Diamino-1,5,8,11-tetramethylundecan, 1,8-Diamino-3,6-dioxaoktan, 1,10-Diamino-4,7-dioxadecan, 1,12-Diamino-4,9-dioxadodecan, 1,14-Diamino-3,10-dioxatetradecan, 1,13-Diamino-4,7,10-trioxatridecanan, 1,7-Diamino-2,6-dioxa-4-aminomethoxy-heptan, 1-Amino-2-oxa-3,3-bis(aminomethoxy)hexan, 1,9-Diamino-3,7-dioxa-5-(1-amino-2-ethoxy)-nonan, 1-Amino-3-oxa-4,4-bis(1-amino-2-ethoxy)heptan, 1,11-Diamino-4,8-dioxa-6-(1-amino-5-oxabutyl)undecan, 1-Amino-4-oxa-5,5-bis(1-amino-5-oxabutyl)oktan oder Mischungen davon. Der limitierende Faktor bezüglich der Verwendbarkeit von Ethergruppen enthaltenden Mono- sowie Polyaminen in der erfindungsgemäßen CoPg in der speziellen Ausführungsfrom der Gasphasenphosgenierung ist weder in der Anzahl der (primären) Aminogruppen noch in der Anzahl der Etherbindungen zu sehen, sondern lediglich durch die Verdampfbarkeit unter dem angewandten Druck gegeben. Bei besonders hochsiedenden Verbindungen kann es deshalb von Vorteil sein, sie als Azeotrope mit anderen Stoffen in die Gasphasenphosgenierung einzubringen oder ein Trägergas für die Zufuhr des Amingemisches in den Reaktionsraum einzusetzen. Hierbei kann Amin 2 unterstützend wirken.

Darüber hinaus ist es bevorzugt, dass der Massenanteil
- des ersten Amins 5,0 bis 95,0 Gew.-%, bevorzugt 10,0 bis 80,0 Gew.-% und
- des zweiten Amins 95,0 bis 5,0 Gew.-%, bevorzugt 90,0 bis 20,0 Gew.-%, weiter bevorzugt 80,0 bis 20,0 beträgt, bezogen auf das Gesamtgewicht des Amin-Gemischs.

Das molare Verhältnis von Phosgen zu den Aminogruppen der Amine des Amin-Gemischs beträgt vorzugsweise ≥ 1:1 bis ≤ 5:1, bevorzugt > 1:1 bis ≤ 3:1, weiter bevorzugt > 1:1 bis ≤ 2:1.

Bei der Umsetzung ist es bevorzugt, wenn zusätzlich ein Inertstoff eingesetzt wird, wobei der Inertstoff ausgewählt ist aus der Gruppe, umfassend oder bestehend aus
- Intergasen, insbesondere Stickstoff, Argon oder Mischung von diesen;
- inerten Lösungsmitteln, insbesondere aromatische Kohlenwasserstoffe, bevorzugt Chlorbenzol, o-Dichlorbenzol, Toluol, Xylol oder Mischungen von diesen;
- oder Mischungen von den vorgenannten Inertgasen und inerten Lösungsmitteln.

Vorzugsweise liegt die Temperatur
- bei der Umsetzung in kondensierter Phase einerseits zwischen -40°C und dem Siedepunkt des verwendeten Lösemittels bzw. niedrigst-siedenden Einsatzstoffes beim in der Reaktion eingestellten Druck und
- bei der Gasphasenphosgenierung zwischen dem Siedepunkt des Eduktes mit dem höchsten Siedepunkt T1 bei Anlagendruck und 600°C, bevorzugt zwischen T1 und 550°C, weiter bevorzugt zwischen T1 und 500°C.

Es ist zudem bevorzugt, dass das zum ersten Amin korrespondierende Isocyanat und/oder das zum zweiten Amin korrespondierende Isocyanat, von dem Reaktionsgemisch abgetrennt wird, wobei die Abtrennung vorzugsweise mittels Destillation, insbesondere Dünnschichtdestillation, Extraktion, Kristallisation, Umkristallisation oder einer Kombination von diesen erfolgt und die jeweiligen Isocyanate getrennt oder als Isocyanat-Gemisch erhalten werden. Weiter bevorzugt ist es, das zum ersten Amin korrespondierende Isocyanat und das zum zweiten Amin korrespondierende Isocyanat, von dem Reaktionsgemisch abgetrennt wird, wobei die Abtrennung vorzugsweise mittels Destillation, insbesondere Dünnschichtdestillation, Extraktion, Kristallisation, Umkristallisation oder einer Kombination von diesen erfolgt und die jeweiligen Isocyanate getrennt oder als Isocyanat-Gemisch erhalten werden. Dabei beträgt der Gehalt an aciden Chlorverbindungen (auch als AC-Wert, AC-Gehalt oder Acidität bezeichnet) in dem/den abgetrennten Isocyanat(en) oder dem Isocyanat-Gemisch von 1 bis 100 ppm, bevorzugt von 2 bis 80 ppm, besonders bevorzugt von 5 bis 50 ppm, bestimmt gemäß der Norm ISO 15028:2014. Der Gehalt an hydrolysierbarem Chlor (auch als HC-Wert oder HC-Gehalt bezeichnet) in dem/den abgetrennten Isocyanat(en) oder dem Isocyanat-Gemisch beträgt bevorzugt von 1 bis 500 ppm, bevorzugt von 5 bis 100 ppm, bestimmt gemäß der Norm ISO 15028:2014.

Die Erfindung betrifft ferner die Verwendung eines Amin-Gemischs aus mindestens einem ersten Amin und mindestens einem von dem ersten Amin unterschiedlichen zweiten Amin in einem Verfahren zur Herstellung der korrespondierenden Isocyanate durch Phosgenierung nach einem Verfahren gemäß einem der Ansprüche 1 bis 13 zur Erniedrigung des Gehaltes an aciden Chlorverbindungen und/oder des Gehaltes an hydrolysierbarem Chlor der bei der Herstellung erhaltenen korrespondierenden Isocyanate.

Ein weiterer Gegenstand der vorliegenden Erfindung ist ein Produkt erhältlich oder erhalten durch das erfindugsgemäße Verfahren, vorzugsweise unmittelbar erhältlich oder erhalten durch das erfindungsgemäße Verfahren.

Gegenstand der vorliegenden Erfindung ist außerdem die Verwendung des erfindungsgemäßen Produktes und/oder des Isocyanats oder Isocyanat-Gemischs erhalten oder erhältlich nach dem erfindungsgemäßen Verfahren als Komponente zur Herstellung von Polyurethanen, insbesondere Polyurethan-Schaumstoffen, Polyurethan-Beschichtungen und Polyurethan-Klebstoffen, von pharmazeutischen Erzeugnissen, insbesondere Wirkstoffen, sowie von Hilfsstoffen, insbesondere von Hilfsstoffen für die Nassfestausrüstung von Papier und anderen Zelluloseprodukten, Emulgatoren und Verdickungsmitteln.

Das erfindungsgemäße GPP-Verfahren wird in seinen Grundzügen mittels einer an sich bekannten Methode des Standes der Technik, z. B. nach der Lehre der EP 0 570 799 oder der EP 0676392 sowie dort zitierter Verfahrensvarianten, durchgeführt. Hierfür werden die Reaktionspartner nahe oder oberhalb der Siedetemperatur des Ausgangsamin(gemisch)es in geeignete Reaktoren eingeführt, vermischt und miteinander umgesetzt. Die Temperaturen hierfür liegen, abhängig vom gewählten Druck, zwischen 100 und 600 °C, vorzugsweise zwischen 150 und 500 °C. Das Verfahren wird in einem Druckbereich zwischen 10 mbar und 5 bar, vorzugsweise 200 mbar und 3 bar durchgeführt. Die Zuführung der Reaktionskomponenten bei der Gasphasenphosgenierung kann mit oder ohne Verwendung, bezüglich der Phosgenierreaktion, inerter Zusatzstoffe wie z.B. Trägergase erfolgen. Als Trägergase sind sowohl Stickstoff, Argon oder andere inerte Gase als auch Dämpfe technisch verfügbarer Lösungsmittel, wie z.B. Chlorbenzol, Dichlorbenzole, Xylole, Chlornaphthaline, Decahydronaphthalin etc., geeignet.

Anschließend werden durch Abkühlung des Gasstromes bis zu einer Temperatur oberhalb der Zersetzungstemperatur der entsprechenden, intermediären Carbamidsäurechloride die erfindungsgemäßen Isocyanatmischungen erhalten In einer bevorzugten Ausführungsform wird dann das zum ersten Amin korrespondierende Isocyanat und /oder das zum zweiten Amin korrespondierende Isocyanat, von dem Reaktionsgemisch abgetrennt, wobei die Abtrennung vorzugsweise mittels Destillation, insbesondere Dünnschichtdestillation, Extraktion, Kristallisation, Umkristallisation oder einer Kombination von diesen erfolgt und die jeweiligen Isocyanate getrennt oder als Isocyanat-Gemisch erhalten werden. Besonders bevorzugt wird das zum ersten Amin korrespondierende Isocyanat und das zum zweiten Amin korrespondierende Isocyanat, von dem Reaktionsgemisch abgetrennt. Es ist ferner bevorzugt, dass die Abtrennung mittels Destilliation optional gefolgt von einer Umkristallisation erfolgt.

Die erfindungsgemäßen Verfahrensprodukte sind wertvolle Rohstoffe zur Herstellung von Polyurethanen, Klebstoffen, Beschichtungsmitteln, oligomeren Isocyanatmodifizierungsprodukten wie Uretdion-, Isocyanurat-, Carbodiimid-, Biuret-, Urethan- sowie Allophanat-haltigen Polyisocyanaten, Hilfsmitteln wie sie z.B. für die Naßfestausrüstung von Papier und anderer Zelluloseprodukte, als Emulgator, Verdickungsmittel u.a. Verwendung finden, als Rohstoff zur Herstellung und/oder Formulierung von Wirkstoffen, Pharmazeutika u.a..

Schließlich betrifft die Erfindung auch eine Phosgenierungsanlage zur Durchführung des erfindungsgemessen Verfahrens, umfassend oder bestehend aus
(a) mindestens eine Phosgen-Vorrichtung zur kontinuierlichen Bereitstellung von Phosgen, gegebenenfalls in Kombination mit einem Inertstoff
(b) gegebenenfalls eine Inertstoff-Vorrichtung zur Bereitstellung eines Inertstoffs,
(c) mindestens eine Amin-Vorrichtung zur kontinuierlichen Bereitstellung eines Amin- Gemischs, gegebenenfalls in Kombination mit einem Inertstoff
(d) einen Phosgenierungsreaktor zur Vermischung des Phosgens und des Amin-Gemischs sowie gegebenenfalls des Inertstoffs und Umsetzung des Amin-Gemischs mit dem Phosgen,
dadurch gekennzeichnet, dass die Amin-Vorrichtung einen ersten Vorlagebehälter für ein erstes Amin und einen zweiten Vorlagebehälter für ein zweites Amin sowie eine Dosiereinrichtung zur unabhängig voneinander variablen Einstellung der Massenanteile des ersten Amins und des zweiten Amins und optional eine Mischeinrichtung zur Vermischung des ersten Amins und des zweiten Amins zu dem Amin-Gemisch umfasst.

Die Phosgenstrom-Vorrichtung, die optionale Inertstoffstrom-Vorrichtung, die Aminstrom-Vorrichtung sowie der Phosgenierungsreaktor können unabhängig voneinander weitere Apparate umfassen, die für den Betrieb der Phosgnierungsanlage dienlich sind, wie beispielsweise Vorlagebehälter für die Ausgangsstoffe, Wärmetauscher zur Temperierung oder gegebenenfalls Verdampfung von Ausgangsstoffen, Einrichtungen zur Aufbereitung der Reaktionsmischung nach erfolgter Umsetzung von Amin mit Phosgen.

Die Dosiereinrichtung kann beispielsweise eine Dosierpumpe oder ein Regelventil sein. Die optionale Mischeinrichtung kann dabei beispielsweise ein T-Stück in der Rohrleitung, eine Mischkammer, ein Statikmischer oder eine Pumpe, insbesondere Mischpumpe, oder Rührbehälter sein. Eine Vermischung der Aminströme aus den Dosiereinrichtungen kann jedoch auch im Verdampfer erfolgen. Ein Verdampfer kommt insbesondere bei flüssigen Aminen zum Einsatz.

Vorzugsweise umfasst die Aminstrom-Vorrichtung in wenigstens einer der aminführenden Rohrleitungen wenigstens eine Flanschverbindung, die zur Aufnahme einer Steckscheibe vorbereitet ist. Auf diese Weise wird es ermöglicht, den Aminstrom durch die entsprechende Leitung durch die Steckscheibe sicher zu unterbinden oder die entsprechende Amin-Leitung sicher zu trennen.

Die Phosgenstrom-Vorrichtung, die optionale Inertstoffstrom-Vorrichtung sowie die Aminstrom-Vorrichtung sind jeweils über mindestens eine Zuleitung mit dem Phosgenierungsreaktor verbunden. Die Verbindung der Zuleitungen mit dem Phosgenierungsreaktor kann dabei beispielsweise über einfache Rohrleitungen, Flanschverbindungen, Glattstrahldüsen, Ringspaltdüsen oder ein Mischelement erfolgen. Die Ringspaltdüse kann dabei mit zwei oder mehr, vorzugsweise zwei koaxial angeordneten Kanälen für den Aminstrom und den Phosgenstrom oder einen Inertgasstrom ausgestattet sein, wobei der Aminstrom und der Phosgenstrom erst im Phosgenierungsreaktor aufeinander treffen. Alternativ, insbesondere in der großtechnischen Anwendung, wird das Amin durch ein Einleitrohr, an dessen Ende sich eine zentrale Düse befindet und welches durch einen Deckel sowie gegebenenfalls Halter/Gleichrichter in Position gehalten wird, dem Phosgenierungsreaktor zugegeben so dass sich um das Einleitrohr im Reaktor ein Ringraum bildet, wobei das Phosgen über ein separaten Einlass in den Ringraum und von dort über den Gleichrichter in den Phosgenierungsreaktor gelangt (siehe hierzu auch EP1362847 A2). Der Phosgenierungsreaktor hat eine Mischzone, wo die Ströme erstmals aufeinandertreffen, sowie eine Reaktionszone, wo die Ströme weiter umgesetzt werden. Die Mischzone und Reaktionszone liegen bevorzugt in einem Apparat vor. Ferner ist es bevorzugt, dass der Phosgenierungsreaktor im Wesentlichen senkrecht aufgestellt ist und dieser bevorzugt am oberen Ende mit der Phosgenstrom-Vorrichtung und der Aminstrom-Vorrichtung verbunden ist.

Außerdem umfasst die Phosgenierungsanlage vorzugsweise mindestens eine Quenche zur Abkühlung und zumindest teilweisen Kondensation des Reaktionsgemischs, welche mit dem Phosgenierungsreaktor, vorzugsweise am unteren Ende, verbunden ist, wobei die Quenche insbesondere zur Einbringung von Chlorbenzol, o-Dichlorbenzol und/oder mindestens eines der in der Phosgenierungsanlage hergestellten Isocyanate ausgebildet ist. Insbesondere ist der Quenche ein Auffangbehälter zugeordnet, wobei der Auffangbehälter bevorzugt in der Quenche eingebunden oder separat mit dieser verbunden ist.

Vorzugsweise umfasst die Amin-Vorrichtung, gegebenenfalls die separate Inertstoff-Vorrichtung und/oder die Phosgen-Vorrichtung einen Wärmetauscher zur Erwärmung des Amin-Gemischs, des Phosgens und/oder gegebenenfalls des Inertstoffs.

### Ausführungsformen:

Die vorliegende Erfindung betrifft insbesondere die folgenden Ausführungsformen:
Nach einer ersten Ausführungsform betrifft die Erfindung ein Verfahren zur Herstellung von Isocyanaten durch Phosgenierung der korrespondierenden Amine, wobei ein Amin-Gemisch aus mindestens einem ersten Amin und mindestens einem von dem ersten Amin verschiedenen zweiten Amin mit Phosgen zu einem Reaktionsgemisch umgesetzt wird, dadurch gekennzeichnet, dass
entweder
   - das erste Amin mindestens eine Ether-Gruppe enthält; und
   - das zweite Amin ausgewählt ist aus der Gruppe, umfassend oder bestehend aus Aminoalkanen mit 5 bis 15 Kohlenwasserstoffatomen, welche keine Ether-Gruppen enthalten;
oder
   - das erste Amin mindestens eine cyclische Ether-Gruppe enthält; und
   - das zweite Amin mindestens eine offenkettige Ether-Gruppe und keine cyclischen Ether-Gruppen enthält.

Nach einer zweiten Ausführungsform betrifft die Erfindung ein Verfahren gemäß Ausführungsform 1, dadurch gekennzeichnet, dass das mindestens eine Ether-Gruppe enthaltende erste Amine eine Struktur der allgemeinen Formel X-(-R¹-NHₘ)ₙ aufweist, wobei
- X für H, NHₘ oder C(R²)ₚ;
- R¹ für, gegebenenfalls substituierte und/oder Heteroatom-aufweisende, aliphatische, (cyclo)aliphatische oder aromatische Reste mit vorzugsweise bis zu 10 Kohlenstoffatomen, wobei R¹ mindestens eine Ethergruppe enthält;
- R² für H, gegebenenfalls substituierte und/oder Heteroatom-aufweisende, aliphatische, (cyclo)aliphatische oder aromatische Reste mit vorzugsweise bis zu 10 Kohlenstoffatomen;
- m für 1 oder 2;
- n für 1, 2 oder 3; und
- p für 1, 2 oder 3
steht.

Nach einer dritten Ausführungsform betrifft die Erfindung ein Verfahren gemäß Ausführungsform 1 oder 2, dadurch gekennzeichnet, dass das mindestens eine Ether-Gruppe enthaltende erste Amin, das mindestens eine cyclische Ether-Gruppe enthaltende erste Amin, das Aminoalkane mit 5 bis 15 Kohlenwasserstoffatome umfassende oder daraus bestehende zweite Amin und/oder das mindestens eine offenkettige Ether-Gruppe und keine cyclischen Ether-Gruppen enthaltene zweite Amin ein Diamin ist.

Nach einer vierten Ausführungsform betrifft die Erfindung ein Verfahren gemäß einer der vorstehenden Ausführungsformen, dadurch gekennzeichnet, dass das mindestens eine Ether-Gruppe enthaltende erste Amin ausgewählt ist aus der Gruppe, umfassend oder bestehend aus (Tetrahydrofuran-2,5-diyl)dimethanamin (TEFUDA), 3,6-diamino-hexahydrofuro[3,2-b]furan (z.B. IDDA und/oder ISODA), 3,6-Bis(aminomethyl)-hexahydrofuro[3,2-b]furan, Furan-2,5-diyldimethanamin, (Methan-2,2-diylbis(tetrahydrofuran-5,2-diyl))dimethanamin, (Methan-2,2-diylbis(furan-5,2-diyl))dimethanamin, (Propan-2,2-diylbis(tetrahydrofuran-5,2-diyl))dimethanamin, (Propan-2,2-diylbis(furan-5,2-diyl))dimethanamin; Di(aminoethyl)ether, insbesondere 2,2'-oxybis(ethan-1-amin), 1,1'-oxybis(propan-2-amin), 2-(2-aminoethoxy)propan-1-amin, 2,2'-oxybis(propan-1-amin) und 2-(2-aminopropoxy)propan-1-amin; Di(aminopropyl)ether oder Mischungen davon und/oder das mindestens eine cyclische Ether-Gruppe enthaltende erste Amin ausgewählt ist aus der Gruppe, umfassend oder bestehend aus (Tetrahydrofuran-2,5-diyl)dimethanamin, 3,6-diamino-hexahydrofuro[3,2-b]furan (z.B. IDDA und/oder ISODA), 3,6-Bis(aminomethyl)-hexahydrofuro[3,2-b]furan, Furan-2,5-diyldimethanamin, (Methan-2,2-diylbis(tetrahydrofuran-5,2-diyl))dimethanamin, (Methan-2,2-diylbis(furan-5,2-diyl))dimethanamin, (Propan-2,2-diylbis(tetrahydrofuran-5,2-diyl))dimethanamin, (Propan-2,2-diylbis(furan-5,2-diyl))dimethanamin oder Mischungen davon.

Nach einer fünften Ausführungsform betrifft die Erfindung ein Verfahren gemäß einer der vorstehenden Ausführungsformen, dadurch gekennzeichnet, dass das Aminoalkan mit 5 bis 15 Kohlenwasserstoffatomen umfassende oder daraus bestehende zweite Amin ausgewählt ist aus der Gruppe, umfassend oder bestehend aus 1,5-Diaminopentan; 1,6-Diaminohexan; 1-Amino-3,5,5-trimethyl-5-aminomethylcyclohexan; Amino-[(aminocyclohexyl)methyl]cyclohexan, insbesondere 4,4'-Methylenbis(cyclohexan-1-amin), 2-((4-Aminocyclohexyl)methyl)cyclohexan-1-amin und 2,2'-Methylenbis(cyclohexan-1-amin); 1,3-Bis(aminomethyl)cyclohexan; 1,4-Bis(aminomethyl)cyclohexan; Diaminocyclohexan, insbesondere Cyclohexan-1,2-diamin, Cyclohexan-1,3-diamin und Cyclohexan-1,4-diamin; Methyl-diamino-cyclohexan, insbesondere 4-Methylcyclohexan-1,3-diamin und 2-Methylcyclohexan-1,3-diamin; 4,4'-methylenbis(2-methylcyclohexan-1-amin); oder Mischungen davon.

Nach einer sechsten Ausführungsform betrifft die Erfindung ein Verfahren gemäß einer der vorstehenden Ausführungsformen, dadurch gekennzeichnet, dass das mindestens eine offenkettige Ether-Gruppen und keine cyclischen Ether-Gruppen enthaltende zweite Amin ausgewählt ist aus der Gruppe, umfassend oder bestehend aus Di(aminoethyl)ether, Di(aminopropyl)ether, 1,8-Diamino-1,5,8-trimethyl-3,6-dioxaoctan, 1,11-Diainino-1,5,8,11-tetramethylundecan, 1,8-Diamino-3,6-dioxaoktan, 1,10-Diamino-4,7-dioxadecan, 1,12-Diamino-4,9-dioxadodecan, 1,14-Diamino-3,10-dioxatetradecan, 1,13-Diamino-4,7,10-trioxatridecanan, 1,7-Diamino-2,6-dioxa-4-aminomethoxy-heptan, 1-Amino-2-oxa-3,3-bis(aminomethoxy)hexan, 1,9-Diamino-3,7-dioxa-5-(1-amino-2-ethoxy)-nonan, 1-Amino-3-oxa-4,4-bis(1-amino-2-ethoxy)heptan, 1,11-Diamino-4,8-dioxa-6-(1-amino-5-oxabutyl)undecan, 1-Amino-4-oxa-5,5-bis(1-amino-5-oxabutyl)oktan oder Mischungen davon.

Nach einer siebten Ausführungsform betrifft die Erfindung ein Verfahren gemäß einer der vorstehenden Ausführungsformen, dadurch gekennzeichnet, dass der Massenanteil
- des ersten Amins 5,0 bis 95,0 Gew.-%, bevorzugt 10,0 bis 80,0 Gew.-% und
- des zweiten Amins 95,0 bis 5,0 Gew.-%, bevorzugt 90,0 bis 20,0 Gew.-%, weiter bevorzugt 80,0 bis 20,0 beträgt, bezogen auf das Gesamtgewicht des Amin-Gemischs.

Nach einer achten Ausführungsform betrifft die Erfindung ein Verfahren gemäß einer der vorstehenden Ausführungsformen, dadurch gekennzeichnet, dass das molare Verhältnis von Phosgen zu den Aminogruppen der Amine des Amin-Gemischs ≥ 1:1 bis ≤ 5:1, bevorzugt > 1:1 bis ≤ 3:1 beträgt.

Nach einer neunten Ausführungsform betrifft die Erfindung ein Verfahren gemäß einer der vorstehenden Ausführungsformen, dadurch gekennzeichnet, dass bei der Umsetzung ein Inertstoff eingesetzt wird, wobei der Inertstoff ausgewählt ist aus der Gruppe, umfassend oder bestehend aus
- Intergasen, insbesondere Stickstoff, Argon oder Mischung von diesen;
- inerten Lösungsmitteln, insbesondere aromatische Kohlenwasserstoffe, bevorzugt Chlorbenzol, o-Dichlorbenzol, Toluol, Xylol oder Mischungen von diesen;
- oder Mischungen von den vorgenannten Inertgasen und inerten Lösungsmitteln.

Nach einer zehnten Ausführungsform betrifft die Erfindung ein Verfahren gemäß einer der vorstehenden Ausführungsformen, dadurch gekennzeichnet, dass die Temperatur
- bei der Umsetzung in kondensierter Phase einerseits zwischen -40°C und dem Siedepunkt des verwendeten Lösemittels bzw. niedrigst-siedenden Einsatzstoffes beim in der Reaktion eingestellten Druck und
- bei der Gasphasenphosgenierung zwischen dem Siedepunkt des Eduktes mit dem höchsten Siedepunkt T1 bei Anlagendruck und 600°C, bevorzugt zwischen T1 und 550°C, weiter bevorzugt zwischen T1 und 500°C liegt.

Nach einer elften Ausführungsform betrifft die Erfindung ein Verfahren gemäß einer der vorstehenden Ausführungsformen, dadurch gekennzeichnet, dass das zum ersten Amin korrespondierende Isocyanat und/oder das zum zweiten Amin korrespondierende Isocyanat, von dem Reaktionsgemisch abgetrennt wird, wobei die Abtrennung vorzugsweise mittels Destillation, insbesondere Dünnschichtdestillation, Extraktion, Kristallisation, Umkristallisation oder einer Kombination von diesen erfolgt und die jeweiligen Isocyanate getrennt oder als Isocyanat-Gemisch erhalten werden.

Nach einer zwölften Ausführungsform betrifft die Erfindung ein Verfahren gemäß Ausführungsform 11, dadurch gekennzeichnet, dass der Gehalt an aciden Chlorverbindungen in dem/den abgetrennten Isocyanat(en) oder dem Isocyanat-Gemisch von 1 bis 100 ppm, bevorzugt von 2 bis 80 ppm, besonders bevorzugt von 5 bis 50 ppm beträgt, bestimmt gemäß der Norm ISO 15028:2014.

Nach einer dreizehnten Ausführungsform betrifft die Erfindung ein Verfahren gemäß einer Ausführungsformen 11 oder 12, dadurch gekennzeichnet, dass der Gehalt an hydrolysierbarem Chlor in dem/den abgetrennten Isocyanat(en) oder dem Isocyanat-Gemisch von 1 bis 500 ppm, bevorzugt von 5 bis 100 ppm beträgt, bestimmt gemäß der Norm ISO 15028:2014.

Nach einer vierzehnten Ausführungsform betrifft die Erfindung die Verwendung eines Amin-Gemischs aus mindestens einem ersten Amin und mindestens einem von dem ersten Amin unterschiedlichen zweiten Amin in einem Verfahren zur Herstellung der korrespondierenden Isocyanate durch Phosgenierung nach einem Verfahren gemäß einer der Ausführungsformen 1 bis 13 zur Erniedrigung des Gehaltes an aciden Chlorverbindungen und/oder des Gehaltes an hydrolysierbarem Chlor der bei der Herstellung erhaltenen korrespondierenden Isocyanate.

Nach einer fünfzehnten Ausführungsform betrifft die Erfindung ein Produkt erhältlich oder erhalten durch ein Verfahren gemäß einer der Ausführungsformen 1 bis 13, vorzugsweise unmittelbar erhältlich oder erhalten durch einer der Ausführungsformen 1 bis 13.

Nach einer sechszehnten Ausführungsform betrifft die Erfindung die Verwendung des Produkts gemäß Ausführungsform 15 und/oder des Isocyanats oder Isocyanat-Gemischs erhalten oder erhältlich nach einem Verfahren gemäß einer der Ausführungsformen 11 bis 13 als Komponente zur Herstellung von Polyurethanen, insbesondere Polyurethan-Schaumstoffen, Polyurethan-Beschichtungen und Polyurethan-Klebstoffen, von pharmazeutischen Erzeugnissen, insbesondere Wirkstoffen, sowie von Hilfsstoffen, insbesondere von Hilfsstoffen für die Nassfestausrüstung von Papier und anderen Zelluloseprodukten, Emulgatoren und Verdickungsmitteln.

Nach einer siebzehnten Ausführungsform betrifft die Erfindung eine Phosgenierungsanlage zur Durchführung des Verfahrens gemäß den Ausführungsformen 1 bis 13, umfassend oder bestehend aus
(a) mindestens eine Phosgen-Vorrichtung zur kontinuierlichen Bereitstellung von Phosgen, gegebenenfalls in Kombination mit einem Inertstoff
(b) gegebenenfalls eine Inertstoff-Vorrichtung zur Bereitstellung eines Inertstoffs,
(c) mindestens eine Amin-Vorrichtung zur kontinuierlichen Bereitstellung eines Amin-Gemischs, gegebenenfalls in Kombination mit einem Inertstoff
(d) einen Phosgenierungsreaktor zur Vermischung des Phosgens und des Amin-Gemischs sowie gegebenenfalls des Inertstoffs und Umsetzung des Amin-Gemischs mit dem Phosgen, dadurch gekennzeichnet, dass die Amin-Vorrichtung einen ersten Vorlagebehälter für ein erstes Amin und einen zweiten Vorlagebehälter für ein zweites Amin sowie eine Dosiereinrichtung zur unabhängig voneinander variablen Einstellung der Massenanteile des ersten Amins und des zweiten Amins und optional eine Mischeinrichtung zur Vermischung des ersten Amins und des zweiten Amins zu dem Amin-Gemisch umfasst.

Nach einer achtzehnten Ausführungsform betrifft die Erfindung eine Vorrichtung gemäß Ausführungsform 17, dadurch gekennzeichnet, dass die Amin-Vorrichtung, gegebenenfalls die separate Inertstoff-Vorrichtung und/oder die Phosgen-Vorrichtung einen Wärmetauscher zur Erwärmung des Amin-Gemischs, des Phosgens und/oder gegebenenfalls des Inertstoffs umfasst.

### Beispiele:

Die nachstehend beschriebenen Beispiele sollen das Verfahren sowie einige der erfindungsgemäßen Verfahrensprodukte näher beschreiben ohne die Erfindung einzuschränken, alle Prozentangaben beziehen sich, wenn nicht anders angegeben, auf die Masse.

Mol%-Angaben wurden ¹H-NMR spektroskopisch ermittelt. Die Messungen wurden auf den Geräten Bruker DPX 400 bzw. DRX 700 an ca. 5%igen (¹H NMR) bzw. ca. 50%igen (¹³C NMR) Proben in trockenem C₆D₆, wenn nicht anders vermerkt, bei 400 bzw. 700 MHz (¹H NMR) oder 100 bzw. 176 MHz (¹³C NMR) durchgeführt. Als Referenz für die ppm-Skale dienten Tetramethylsilan im Lösemittel ¹H NMR chemical shift 0 ppm. Alternativ wurde im NMR-Lösemittel anwesendes C₆D₅H als Referenzsignal (7,15 ppm, ¹H-NMR) verwendet bzw. das Lösemittelsignal selbst (mittleres Signal des 1:1:1 Tripletts bei 128,0 ppm in der ¹³C NMR. ¹⁵N-NMR chemische Verschiebungen wurden indirekt mittels ¹H-¹⁵N-HMBC-Messungen ermittelt, wobei extern auf (flüssiges) Ammoniak referenziert wurde (0 ppm).

Dynamische Viskositäten wurden bei 23°C mit dem MCR 501-Rheometer (Fa. Anton Paar) gemäß DIN EN ISO 3219:1994-10 bestimmt. Durch Messung bei unterschiedlichen Scherraten wurde sichergestellt, dass von newtonischem Fließverhalten ausgegangen werden kann. Angaben zur Scherrate können daher entfallen.

Der NCO-Gehalt wurde durch Titration gemäß DIN EN ISO 10283:2007-11 ermittelt.

Der Restmonomerengehalt wurde gaschromatographisch nach DIN EN ISO 10283:2007-11 mit internem Standard bestimmt.

Der Gehalt an aciden Chlorverbindungen und der Gehalt an hydrolysierbarem Chlor wurde gemäß der Norm ISO 15028:2014 bestimmt.

GC-MS wurde mit dem Agilent GC6890, ausgestattet mit einer MN 725825.30 Optima-5-MS-Accent Kapilllarsäule (30 m, 0,25 mm Innendurchmesser, 0,5 µm Filmschichtdicke) und einem Massenspektrometer *5973* als Detektor mit Helium als Transportgas (Flussrate 2 ml/min) durchgeführt. Die Säulentemperatur betrug anfangs 60 °C (2 min) und wurde anschließend schrittweise mit 8K/min auf 360 °C erhöht. Für die GC-MS Detektion wurde Elektronenstoßionisation mit 70 eV Ionisationsenergie benutzt. Als Injektortemperatur wurde 250 °C gewählt.

Größenausschlußchromatographie (engl.: *Size Exclusion Chromatography, SEC*) wurde gemäß DIN 55672-1:2016-03 mit Tetrahydrofuran als Elutionsmittel durchgeführt.

Röntgenkristallstrukturanalyse erfolgte an einem Oxford Diffraction Xcalibur ausgestattet mit einem CCD-Flächendetektor (Model Ruby), einer Cu_{Kα} Quelle und Osmic Spiegeln als Monochromator bei 106-107 K.

Das Program CrysAlis Version 1.171.38.43 (Rigaku 2015) wurde für die Datenaquisition und -reduktion verwendet. SHELXTL Version 6.14 (Bruker AXS, 2003) fand Einsatz für die Strukturlösung.

Die Messung der Hazen-Farbzahl erfolgte spektrophotometrisch nach DIN EN ISO 6271-2:2005-03 mit einem LICO 400-Spektrofotometer der Fa. Lange, DE.

Alle Reaktionen wurden unter einer Stickstoffatmosphäre in vorher im Vakuum bei 150 - 200°C getrockneten Glasapparaturen durchgeführt.

Die verwendeten Diisocyanate sind Produkte der Fa. Covestro. Alle anderen kommerziell zugänglichen Chemikalien wurden von der Fa. Aldrich, D-82018 Taufkirchen, bezogen.

1,5-Diamino-3-oxapentan (OBDA) wurde von der Fa. BASF, SE, bezogen. TEFUDA, (3R,3aR,6S,6aR)-hexahydrofuro[3,2-b]furan-3,6-diamin (ISODA) sowie (3S,3aR,6S,6aR)-hexahydrofuro[3,2-b]furan-3,6-diamin (IDDA) wurden aus den entsprechenden Hydroxylverbindungen in Anlehnung an eine Literaturmethode: S. Thiyagarajan, L. Gootjes, W. Vogelzang, J. van Haveren, M. Lutz, D. S. van Es ChemSusChem 2011, 4, 1823 - 1829) hergestellt.

### Allgemeine Arbeitsvorschriften

### A Phosgenierungen in der Flüssigphase (FPP)

In einem auf - 5°C gekühlten 4-Halskolben mit mechanischem Rührer, Tropftrichter, Innenthermometer und Gaseinleitungsrohr wurden 500 ml Monochlorbenzol (MCB) vorgelegt, die doppelt molare Menge Phosgen, bezogen auf umzusetzendes Diamin(gemisch), einkondensiert und bei weiterer Kühlung auf -10 bis maximal 0°C die Lösung des jeweiligen Amin(gemisch)es in MCB langsam zugetropft. Nach vollständiger Amin(gemisch)zugabe wurde der Tropftrichter mit 100 ml MCB beaufschlagt und dieses ebenfalls zur Reaktionsmischung getropft. Unter Phosgeneinleitung (ca. 120 g/h) über das Gaseinleitungsrohr wurde die Temperatur anschließend schrittweise erhöht bis MCB-Rückfluss eintrat. Nach Erreichen der Siedetemperatur wurde unter weiterer Phosgendurchleitung (ca. 120 g/h) so lange am Rückfluß nachgerührt, bis eine klare Lösung entstanden war, mindestens aber 5 Stunden. Zur Entphosgenierung wurde weiter am Rückfluß nachgerührt und über das Gaseinleitungsrohr statt Phosgen trockener Stickstoff durchgeleitet, bis mittels Phosgenindikatorpapier Phosgenfreiheit sichergestellt war. Die weitere Aufarbeitung ist bei den jeweiligen Beispielen beschrieben.

### B Phosgenierungen in der Gasphase (GPP)

In einer Glasanlage mit beheizbarem Mischrohr von 2,5 mm Durchmesser und 17,5 mm Länge mit nachgeschalteter Kondensationsstufe und anschließendem, Aktivkohle-gefüllten Phosgen-Adsorptionsturm, wurde kontinuierlich durch eine in das Mischrohr ragende Düse Phosgen eingeleitet, das in einem vorgeschalteten Wärmetauscher vorerhitzt wurde. Durch den Ringspalt zwischen Düse und Mischrohr wurde simultan mit einer je nach Amin(gemisch) optimierten Dosiergeschwindigkeit ein ebenfalls vorerhitztes Amingemisch, optional verdünnt mit ebenfalls vorerhitztem, trockenen Stickstoff als Verdünnungsmittel, in den Reaktionsraum eingeleitet. Durch Anlegen eines Unterdruckes am Ende der Kondensationsstufe wurde im Mischrohr ein definierter Druck aufrecht erhalten. Das heiße, den Reaktionsraum gasförmig verlassende Reaktionsgemisch wurde in einer Kondensationsstufe durch am Rückfluß siedendes 1,2-Dichlorbenzol geleitet. Hierbei erfolgte die selektive Kondensation der gebildeten Diisocyanate. Das die Waschstufe durchlaufende, im wesentlichen aus Stickstoff, HCl und überschüssigem Phosgen bestehende Gasgemisch wurde in dem Adsorptionsturm anschließend vom Phosgen befreit und das Kondensat wie unter A beschrieben von restlichem, gelöstem Phosgen befreit. Die weitere Aufarbeitung ist bei den jeweiligen Beispielen beschrieben.

### Beispiel 1 (Vergleichsversuche)

a) Phosgenierung von OBDA in der Flüssigphase,
b) Cophosgenierung von OBDA mit MDA gemäß DE 2249459 und
c) Cophosgenierung von IDDA mit MDA gemäß DE 2249459
   **a)**
      20,83 g (0,2 mol) OBDA wurden in ca. 400 ml MCB gelöst und gemäß allg. Arbeitsvorschrift A phosgeniert. Die so erhaltene Reaktionsmischung wurde mittels GC / GC-MS untersucht. Neben dem Lösemittel wurden 3 Hauptkomponenten im Verhältnis 3 : 1 : 47 zueinander detektiert: 2-Chlorethylisocyanat, 2-(2-Chlorethoxy)ethylisocyanat und OBDI. Nach Niedrigsiederabtrennung (Monochlorbenzol und 2-Chlorethylisocyanat) über eine 40 cm lange Füllkörperkolonne, Innendurchmesser ca. 25 mm, Füllung: #1 Interpack bei reduziertem Druck wurde der verbliebene, weitgehend lösemittelfreie Destillationsrückstand anschließend sofort einer "trap-to-trap" Destillation bei 0,1 mbar und 120 - 180°C Badtemperatur unterworfen. Dabei resultierten 10,5 g eines dunkelbraunen, hochviskosen Destillationsrückstandes mit 10,8% NCO-Gehalt und 15,2 g eines farbhellen Destillates mit 53,0% NCO- und 220/560 ppm AC/HC-Gehalt welches lt. NMR und GC-MS zu ca. 98% aus OBDI und ca. 2% aus 2-(2-Chlorethoxy)ethylisocyanat bestand (ca. 50% Ausbeute an OBDI bezogen auf eingesetztes Amin). Nach einigen Tagen verfärbte sich die vorher nahezu farblose Flüssigkeit deutlich. Das Produkt ist für den weiteren problemlosen Einsatz in typischen Polyurethananwendungen ungeeignet.
   **b)**
      Eine Mischung aus 4,17 g (0,04 mol) OBDA und 31,78 g MDA wurden in ca. 400 ml Monochlorbenzol gelöst und gemäß allg. Arbeitsvorschrift A phosgeniert. Die so erhaltene Reaktionsmischung wurde mittels GC / GC-MS untersucht. Neben dem Lösemittel und aromatischen Isocyanaten wurden 3 Hauptkomponenten im Verhältnis 8,5 : 1 : 139 zueinander detektiert: 2-Chlorethylisocyanat, 2-(2-Chlorethoxy)ethylisocyanat und OBDI. Nach Niedrigsiederabtrennung (Monochlorbenzol und 2-Chlorethylisocyanat) über eine 40 cm lange Füllkörperkolonne, Innendurchmesser ca. 25 mm, Füllung: #1 Interpack bei reduziertem Druck wurde der verbliebene, weitgehend lösemittelfreie Destillationsrückstand anschließend sofort einer "trap-to-trap" Destillation bei 0,1 mbar und 120 - 180°C Badtemperatur unterworfen. Dabei resultierten 29,5 g eines dunkelbraunen, hochviskosen Destillationsrückstandes mit 26,8 % NCO-Gehalt der nicht weiter analysiert wurde (größtenteils polmer-MDI) und 12,7 g eines gelblichen Destillates mit 40,0% NCO-Gehalt, welches lt. NMR und GC-MS zu ca. 31,3 % aus OBDI, ca. 0,2 % aus 2-(2-Chlorethoxy)ethylisocyanat und ca. 68,1 % 2-Kern-MDI (3 verschiedene Isomere, hauptsächlich 4,4'-Isomer) neben weiteren, nicht identifizierten Verunreinigungen bestand (ca. 64 % Ausbeute an OBDI bezogen auf eingesetztes OBDA). Der hohe Anteil an Spaltprodukten (2-Chlorethylisocyanat, 2-(2-Chlorethoxy)ethylisocyanat) und die niedrige Ausbeute an OBDI belegen, dass die Verfahrensweise gemäß DE 2249459 bei der Verwendung des ersten Amins (Amin 1) nicht zum Erfolg führt.
   **c)**
      Eine Mischung aus 5,8 g (0,04 mol) IDDA und 32,0 g MDA wurden in ca. 1000 ml Monochlorbenzol gelöst und gemäß allg. Arbeitsvorschrift A phosgeniert. Die so erhaltene Reaktionsmischung wurde am Rotationsverdampfer bei reduziertem Druck weitgehend vom Lösemittel befreit und anschließend sofort einer "trap-to-trap" Destillation bei 0,1 mbar und 120 - 180°C Badtemperatur unterworfen. Dabei resultierten 20,8 g eines dunkelbraunen, hochviskosen Destillationsrückstandes mit 28,0 % NCO-Gehalt der nach Ausweis der GPC und NMR größtenteils aus polmer-MDI bestand und 27,6 g eines gelblichen Destillates welches lt. NMR und GC-MS zu ca. 25 % aus IDDI und ca. 75 % aus 2-Kern-MDI (3 verschiedene Isomere, hauptsächlich 4,4'-Isomer) neben weiteren, nicht identifizierten Verunreinigungen bestand. Eine destillative Auftrennung dieses Gemisches gelang nicht. Es verblieben selbst nach mehrfacher Umkristallisation aus heißem iso-Oktan Reste des aromatischen Diisocyanates im IDDI. Die Verfahrensweise gemäß DE 2249459 führt bei der Verwendung des ersten Amins (Amin 1) nicht zum Erfolg.

### Beispiel 2 (Vergleichsversuch) Herstellung von IDDI aus IDDA durch FPP ohne das zweite Amin

28,5 g (0,2 mol) IDDA wurden gemäß allg. Arbeitsvorschrift A phosgeniert. Die so erhaltene Reaktionsmischung wurde am Rotationsverdampfer bei reduziertem Druck weitgehend vom Lösemittel befreit und anschließend sofort einer "trap-to-trap" Destillation bei 0,1 mbar und 120 - 180°C Badtemperatur unterworfen. Dabei resultierten 12,2 g eines dunkelbraunen, hochviskosen Destillationsrückstandes und 21,3 g eines farbhellen, nach einiger Zeit kristallisierenden Destillates welches nach Ausweis kombinierter analytischer Methoden (NMR, GC-MS) zu ca. 98% aus IDDI bestand (53% Ausbeute bezogen auf eingesetztes Amin, AC/HC: 616/758 ppm). Nach einigen Tagen der Lagerung bei Zimmertemperatur unter Luftausschluß verfärbte sich der vorher nahezu farblose Feststoff deutlich. Das Produkt ist für den weiteren problemlosen Einsatz in typischen Polyurethananwendungen ungeeignet.

### Beispiel 3 (erfindungsgemäß): CoPg-FPP von IDDA (erstes Amin) und PACM 20 (zweites Amin)

33,7 g (0,16 mol) PACM 20 und 5,8 g (0,04 mol) IDDA wurden gemäß allg. Arbeitsvorschrift A phosgeniert. Die so erhaltene Reaktionsmischung wurde am Rotationsverdampfer bei reduziertem Druck weitgehend vom Lösemittel befreit und anschließend sofort einer "trap-to-trap" Destillation bei 0,1 mbar und 120 - 180°C Badtemperatur unterworfen. Dabei resultierten lediglich 6,2 g eines dunkelbraunen, hochviskosen Destillationsrückstandes und 45,7 g eines farbhellen, nach einiger Zeit kristalline Anteile abscheidenden Destillates welches nach Ausweis kombinierter analytischer Methoden (NMR, GC-MS) zu ca. 13% aus IDDI sowie 84% aus H₁₂MDI bestand (78% sowie 91% Ausbeute bezogen auf eingesetzte Amine). Anschließende Fraktionierung lieferte 5,6 g farbloses, rasch kristallisierendes Destillat (Siedepunkt 83°C bei 0,1 mbar, Schmelzpunkt 42°C) welches auch nach wochenlanger Lagerung bei Zimmertemperatur unter Luftausschluß keine Verfärbungstendenz aufwies und sich für den weiteren problemlosen Einsatz in typischen Polyurethananwendungen als gut geeignet erwies.

### Beispiel 4 (Vergleichsversuch) Herstellung von ISODI aus ISODA durch FPP ohne "zweites Amin"

28,8 g (0,2 mol) ISODA wurden gemäß allg. Arbeitsvorschrift A phosgeniert. Die so erhaltene Reaktionsmischung wurde am Rotationsverdampfer bei reduziertem Druck weitgehend vom Lösemittel befreit und anschließend sofort einer "trap-to-trap" Destillation bei 0,1 mbar und 120 - 180°C Badtemperatur unterworfen. Dabei resultierten 15,1 g eines dunkelbraunen, hochviskosen Destillationsrückstandes und 26,0 g (66% Ausbeute bezogen auf eingesetztes Amin) eines farbhellen Destillates welches nach Ausweis kombinierter analytischer Methoden (NMR, GC-MS) zu ca. 98 % aus ISODI bestand. Der titrierte NCO-Gehalt belief sich auf 42,2% und die AC/HC-Werte auf 904/1548 ppm. Nach einigen Tagen verfärbte sich das vorher nahezu farblose Destillat deutlich und war nach mehreren Wochen der Lagerung bei Zimmertemperatur unter Luftausschluß dunkelbraun. Das Produkt ist für den weiteren problemlosen Einsatz in typischen Polyurethananwendungen ungeeignet.

### Beispiel 5 (erfindungsgemäß): CoPg-FPP von IDDA (erstes Amin) und IPDA (zweites Amin)

5,8 g (0,04 mol) IDDA und 33,7 g (0,16 mol) IPDA wurden gemäß allg. Arbeitsvorschrift A phosgeniert. Die so erhaltene Reaktionsmischung wurde am Rotationsverdampfer bei reduziertem Druck weitgehend vom Lösemittel befreit und anschließend sofort einer "trap-to-trap" Destillation bei 0,1 mbar und 120 - 180°C Badtemperatur unterworfen. Dabei resultierten lediglich 3,6 g eines dunkelbraunen, hochviskosen Destillationsrückstandes und 40,6 g eines farbhellen Destillates welches nach Ausweis kombinierter analytischer Methoden (NMR, GC-MS) zu ca. 16,7 % aus ISODI sowie 83,3 % aus IPDI bestand (86% sowie 95% Ausbeute bezogen auf eingesetzte Amine). Der AC/HC-Gehalt dieser Mischung lag bei 42/132 ppm.

### Beispiel 6 (Vergleichsversuche) Herstellung von H₁₂MDI aus PACM 20 durch GPP gemäß allg. Arbeitsvorschrift B (Versuche a und b) sowie IPDI aus IPDA (Versuch c)

a) In einem auf 350°C vorgeheizten Gasphasenrohrreaktor wurden innerhalb von 2 Stunden 141 g PACM 20 (0,67 mol) mit 580 g Phosgen (5,86 mol) bei 500 mbar zur Reaktion gebracht. Beide Stoffströme wurden jeweils bei gleichem Druck in einem Wärmetauscher verdampft und auf 350°C erhitzt. Zur besseren Aminverdampfung wurden durch den Aminverdampfer 60 1/h Stickstoff als Trägergasstrom geleitet. Unmittelbar nach dem Reaktionsstart wurde deutlich die Bildung von Krusten und Belägen am Amineintritt und im Reaktionsrohr beobachtet. Die genaue Auswaage nach dem Versuch ergab 8 g Feststoff.
b) In einem auf 450°C vorgeheizten Gasphasenrohrreaktor wurden innerhalb von 2 Stunden 139 g PACM 20 (0,66 mol) mit 581 g Phosgen (5,87 mol) bei 500 mbar zur Reaktion gebracht. Beide Stoffströme wurden jeweils bei gleichem Druck in einem Wärmetauscher verdampft und auf 450°C erhitzt. Zur besseren Aminverdampfung wurden durch den Aminverdampfer 60 1/h Stickstoff als Trägergasstrom geleitet. Es wurde im Vergleich zu Beispiel 6a deutlich weniger Krusten und Belege gebildet. Im Reaktor konnte nach dem Versuch nur ein hauchdünner Wandbelag festgestellt werden, welcher durch Rückwägung mit 1,3 g bestimmt wurde.
c) In einem auf 350°C vorgeheizten Gasphasenrohrreaktor wurden innerhalb von 2 Stunden 170 g IPDA (1,0 mol) mit 622 g Phosgen (6,29 mol) bei 500 mbar zur Reaktion gebracht. Beide Stoffströme wurden jeweils bei 500 mbar in einem Wärmetauscher verdampft und auf 350°C erhitzt. Zur besseren Aminverdampfung wurden 30 1/h Stickstoff durch den Aminverdampfer geleitet. Nach dem Reaktionsstart wurde nur eine geringe Bildung von Krusten und Belägen am Amineintritt und im Reaktionsrohr beobachtet, welche nach dem Versuch mit 0,5 g bestimmt wurden.

Die unter a und b erhaltenen Reaktionsmischungen wurden mittels ¹H-NMR untersucht, wobei sich herausstellte, dass neben dem Lösemittel und H₁₂MDI relevante Anteile eines Olefines enthalten waren, welches durch GC-MS als Cyclohexenyl-methyl-cyclohexylisocyanat (mehrere Isomere) identifiziert werden konnte. Im Beispiel 6b fiel der Anteil dieses unerwünschten Nebenproduktes, bezogen auf die Zielverbindung H₁₂MDI, deutlich geringer aus als bei 6a: 67:1 ggü. 4:1 (Masse H₁₂MDI : Masse Olefin).

Nach Lösemittelbefreiung und anschließender "trap-to-trap" Destillation wie bei den Beispielen 2-5 beschrieben resultierten bei a) 6,15 g eines dunkelbraunen, viskosen Destillationsrückstandes und 140,4 g eines farbhellen Destillates welches nach anschließender Fraktionierung über eine 40 cm lange, Vakuumummantelte Vigreuxkolonne mit ca. 25 mm Innendurchmesser 123,6 g eines konstant siedenden, farblosen Hauptlaufes mit nur 30,3% NCO-Gehalt lieferte (<70% Ausbeute an H₁₂MDI auf eingesetztes PACM 20 bezogen). Bei b) fielen 2,8 g Rückstand und 163 g finales Destillat mit 31,8 % NCO-Gehalt an (93 % Ausbeute an H₁₂MDI auf eingesetztes PACM 20 bezogen).

### Beispiel 7 (erfindungsgemäß): CoPg-GPP von TEFUDA (erstes Amin bzw. Amin 1) und PACM 20 (zweites Amin bzw. Amin 2) durch GPP gemäß allg. Arbeitsvorschrift B

In Anlehnung an das Beispiel 6b wurden gemäß der allg. Arbeitsvorschrift B Mischungen aus TEFUDA (Amin 1) und PACM 20 (Amin 2) phosgeniert. Hierbei wurde zum Versuchsstart reines Amin 2 (~0,335 mol/h) mit einem molaren Phosgenüberschuss von etwa 250% dosiert. Nach 30 Minuten Versuchszeit wurde mit Amin 1 so ergänzt, dass eine Aminmischung bestehend aus 25 mol-% Amin 1 und 75 mol-% Amin 2 dosiert wurde. In weiteren Versuchen wurde die Konzentration an Amin 1 weiter erhöht, so dass Aminmischungen mit 50 mol-% Amin 1/50 mol-% Amin 2 sowie 75 mol-% Amin 1/25 mol-% Amin 2 eingesetzt wurden. In Summe wurden 782,6 g (6 mol) TEFUDA und 1431,1 g (6,8 mol) PACM 20 verarbeitet. Die dosierten Mengen, verwendeten Phosgenüberschüsse und Laufzeiten können nachfolgender Tabelle entnommen werden.

| **Beispiel** | **Amin 1** | **Amin 2** | **Aminmenge** | **Phosgenuberschuss** | **Druck** | **Temperatur [°C]** | | | **Laufzeit** | **Reaktor-belegung** |
|---|---|---|---|---|---|---|---|---|---|---|
| | **[mol-%]** | **[mol-%]** | **[mol/h]** | **[mol-%]** | **[mbara]** | **Amin** | **Phosgen** | **Reaktor** | **[h]** | **[g]** |
| 7-1 | 25 | 75 | 0,36 | 245 | 500 | 450 | 450 | 450 | 4,7 | 1,7 |
| 7-2 | 50 | 50 | 0,35 | 252 | 500 | 450 | 450 | 450 | 5,8 | 2,3 |
| 7-3 | 75 | 25 | 0,33 | 267 | 500 | 450 | 450 | 450 | 4,0 | 2,7 |
| **7-4** | 50 | 50 | 0,30 | 292 | 500 | 450 | 450 | 450 | 5,8 | 1,3 |
| **7-5** | 50 | 50 | 0,37 | 242 | 500 | 450 | 450 | 450 | 6,0 | 1,0 |
| **7-6** | 75 | 25 | 0,31 | 300 | 500 | 450 | 450 | 450 | 5,3 | 4,0 |
| **7-7** | 75 | 25 | 0,36 | 238 | 500 | 450 | 450 | 450 | 4,8 | 3,3 |

Die entphosgenierten Rohwaren wurden mittels ¹H-NMR-Spektroskopie untersucht und anschließend vereinigt. Hinsichtlich des H₁₂MDI : Olefin Verhältnisses zeigte sich, das der Anteil der unerwünschten Verbindung gegenüber dem besseren Vergleichsbeispiel 6b in allen Versuchläufen 7-1 bis 7-7 nochmals deutlich verringert wurde und sich im Mittel nur noch auf 146 : 1 belief. Wesentliche Unterschiede bezüglich dieses Verhältnisses wurden in den Versuchsläufen 7-1 bis 7-7 nicht beobachtet.

Aufarbeitung wie unter Beispiel 6 beschrieben, mit dem Unterschied, dass zur finalen Auftrennung von TEFUDI und H₁₂MDI eine wirksamere Kolonne verwendet wurde (40 cm Füllkörperkolonne, Innendurchmesser ca. 25 mm, Füllung: #1 Interpack) lieferte lediglich 53,9 g eines dunkelbraunen, hochviskosen Destillationsrückstandes und 980,5 g TEFUDI sowie 1674,2 g H₁₂MDI jeweils als farbhelle Flüssigkeiten, die nach Ausweis kombinierter analytischer Methoden (NMR, GC-MS) zu >99% aus TEFUDI bzw. H₁₂MDI (Isomerengemisch) bestanden (90 % bzw. 94 % Ausbeute bezogen auf die eingesetzten Amine). Der AC/HC-Gehalt lag bei 35/318 ppm (TEFUDI) bzw. 29/87 ppm (H₁₂MDI). Eine Verfärbung wurde auch nach wochenlanger Lagerung unter Luftabschluß nicht beobachtet.

### Beispiel 8 (erfindungsgemäß): CoPg-GPP von ISODA (erstes Amin bzw. Amin 1) und PACM 20 (zweites Amin bzw. Amin 2) durch GPP gemäß allg. Arbeitsvorschrift B.

In Anlehnung an die Vorgehensweise aus Beispiel 7 wurden in einer Gasphasenphosgenierapparatur Mischungen aus ISODA (Amin 1) und PACM 20 (Amin 2) phosgeniert. Im Versuch 8-1 wurde zum Versuchsstart reines Amin 2 (~0,5 mol/h) bei ca. 250% an molarem Phosgenüberschuss dosiert. Nach 30 Minuten Versuchszeit wurde mit Amin 1 so ergänzt, dass eine Aminmischung bestehend aus 25 mol-% Amin 1 und 75 mol-% Amin 2 dosiert wurde. Diese schrittweise Erhöhung um 25 mol-% wurde jeweils nach weiteren 30 Minuten insgesamt zweimal durchgeführt, so dass 2 Stunden nach Versuchstart eine Aminmischung aus 75 mol-% Amin 1 und 25 mol-% Amin 2 dosiert wurde, die dann bis zum Versuchsende nach 6,25 Stunden Gesamtlaufzeit konstant gehalten wurde. In den nachfolgenden Versuchen wurde nach der ersten Phase mit reinem Amin 2 sofort eine Aminmischung bestehend aus 50 mol-% Amin 1 und 50 mol-% Amin 2 dosiert, welche bis zum Versuchsende beibehalten wurde. Zusätzlich wurde von Versuch zu Versuch der Phosgenüberschuss reduziert, was tendenziell mit einer geringeren Reaktorbelegung einherging. Die in den Einzelversuchen dosierten Mengen, verwendeten Phosgenüberschüsse und Laufzeiten können der nachfolgenden Tabelle entnommen werden. In Summe wurden 1353,9 g (9,39 mol) ISODA und 915,0 g (4,35 mol) PACM 20 verarbeitet.

| **Beispiel** | **Amin 1** | **Amin 2** | **Amin-menge** | **Phosgenuberschuss** | **Druck** | **Temperatur [°C]** | | | **Laufzeit** | **Reaktor-belegung** |
|---|---|---|---|---|---|---|---|---|---|---|
| | **[mol-%]** | **[mol-%]** | **[mol/h]** | **[mol-%]** | **[mbara]** | **Amin** | **Phosgen** | **Reaktor** | **[h]** | **[g]** |
| **8-1** | 75 | 25 | 0,42 | 265 | 500 | 450 | 450 | 450 | 6,3 | 2,6 |
| **8-2** | 75 | 25 | 0,41 | 230 | 500 | 450 | 450 | 450 | 6,8 | 1,8 |
| **8-3** | 75 | 25 | 0,49 | 160 | 500 | 450 | 450 | 450 | 7,5 | 1,6 |
| **8-4** | 75 | 25 | 0,53 | 139 | 500 | 450 | 450 | 450 | 6,0 | 0,7 |
| **8-5** | 75 | 25 | 0,53 | 137 | 500 | 450 | 450 | 450 | 4,3 | 0,8 |

Die entphosgenierten Rohwaren wurden mittels ¹H-NMR-Spektroskopie untersucht und anschließend vereinigt. Hinsichtlich des H₁₂MDI : Olefin Verhältnisses zeigte sich, das der Anteil der unerwünschten Verbindung gegenüber dem besseren Vergleichsbeispiel 6b nochmals deutlich verringert wurde und sich im Mittel nur noch auf 127 : 1 belief. Auch hier wurden keine wesentlichen Unterschiede bezüglich dieses Verhältnisses in den Versuchsläufen 8-1 bis 8-5 beobachtet.

Aufarbeitung wie unter Beispiel 7 beschrieben lieferte lediglich 60,1 g eines dunkelbraunen, hochviskosen Destillationsrückstandes sowie 1795,8 g ISODI sowie 993,1 g H₁₂MDI als farbhelle Flüssigkeiten, die nach Ausweis kombinierter analytischer Methoden (NMR, GC-MS) zu >99% aus ISODI bzw. H₁₂MDI (Isomerengemisch) bestanden (97,5% sowie 87,0% Ausbeute bezogen auf die eingesetzten Amine). Der AC/HC-Gehalt lag bei 27/178 ppm (ISODI) bzw. 15/152 ppm (H₁₂MDI). Eine Verfärbung wurde bei beiden erfindungsgemäß erhaltenen Produkten auch nach wochenlanger Lagerung bei Zimmertemperatur unter Luftabschluß nicht beobachtet.

### Beispiel 9

**a)** Zwei Vergleichsversuche GPP von OBDA
**b)** erfindungsgemäß: CoPg-GPP von OBDA (erstes Amin) und IPDA (zweites Amin) durch GPP gemäß allg. Arbeitsvorschrift B

### a)

In zwei separaten Versuchen wurden in einem auf 350°C vorgeheizten Gasphasenrohrreaktor OBDA und Phosgen bei 500 mbar zur Reaktion gebracht. Beide Stoffströme wurden jeweils bei 500 mbar in einem Wärmetauscher verdampft und auf 350°C erhitzt. Zur besseren Aminverdampfung wurden durch den Aminverdampfer 30 1/h Stickstoff als Trägergas geleitet. Im ersten Vergleichsversuch wurden innerhalb von 3,8 Stunden 325 g OBDA (3,1 mol) mit 1376 g Phosgen (13,9 mol) umgesetzt. Im zweiten Vergleichsversuch wurden innerhalb von 3,5 Stunden 312 g OBDA (3,0 mol) und 1219 g Phosgen (12,3 mol) dosiert, wobei nach dem Versuch die Reaktorbelegung mit 9 g bestimmt wurde.

Aufarbeitung wie unter Beispiel 6 beschrieben lieferte 185 g eines dunkelbraunen, hochviskosen Destillationsrückstandes und 723,0 g OBDI als farbhelle Flüssigkeit die nach Ausweis kombinierter analytischer Methoden (NMR, GC-MS) zu >98% aus OBDI bestand (76 % Ausbeute bezogen auf eingesetztes Amin). Der AC/HC-Gehalt lag bei 90/793 ppm. Im Verlaufe mehrerer Wochen der Lagerung bei Zimmertemperatur unter Luftabschluß verfärbte sich das Produkt deutlich. Die Farbzahl stieg von anfangs 8 Apha unmittelbar nach der destillativen Aufarbeitung auf 520 Apha nach 3 Wochen an.

### b)

In Anlehnung an das Beispiel 6c wurde in einen auf 350°C vorgeheizten Gasphasenrohrreaktor innerhalb von 6 Stunden eine Aminmischung bestehend aus 118,4 g OBDA (1,14 mol) und 355,3 g IPDA (2,09 mol) dosiert und mit 2223 g Phosgen (22,47 mol) bei 500 mbar Anlagendruck zur Reaktion gebracht. Beide Stoffströme wurden jeweils bei 500 mbar in einem Wärmetauscher verdampft und auf 350°C erhitzt. Zur besseren Aminverdampfung wurden 30 1/h Stickstoff durch den Aminverdampfer geleitet. Hinter dem Reaktor wurde die Reaktionsmischung in siedendem o-Dichlorbenzol gequencht und mittels Stickstoffstrippung entphosgeniert. Die Feststoffbildung am Amineintritt und im Reaktionsrohr wurde nach dem Versuch mit 4 g bestimmt.

Aufarbeitung wie unter Beispiel 6 beschrieben lieferte lediglich 12 g eines dunkelbraunen, hochviskosen Destillationsrückstandes, 169 g OBDI sowie 442 g IPDI als farbhelle Flüssigkeiten, die nach Ausweis kombinierter analytischer Methoden (NMR, GC-MS) zu >99% aus OBDI bzw. IPDI (Isomerengemisch) bestanden (jeweils 95% Ausbeute bezogen auf die eingesetzten Amine). Der AC/HC-Gehalt lag bei 19/114 ppm (OBDI) bzw. 12/212 ppm (IPDI). Eine Verfärbung wurde bei beiden erfindungsgemäß erhaltenen Produkten auch nach wochenlanger Lagerung bei Zimmertemperatur unter Luftabschluß nicht beobachtet.

### Beispiel 10 (erfindungsgemäß): CoPg-GPP von ISODA (erstes Amin) und OBDA (zweites Amin)

In einen auf 350°C vorgeheizten Gasphasenrohrreaktor wurden anfangs 42,2 g OBDA (0,4 mol) und 152 g Phosgen (1,54 mol) innerhalb von 30 Minuten dosiert. Anschliessend wurde innerhalb von 3 Stunden eine Aminmischung bestehend aus 54,0 g ISODA (0,37 mol) und 117,3 g OBDA (1,13 mol) dosiert und mit 933 g Phosgen (9,43 mol) bei 500 mbar Anlagendruck zur Reaktion gebracht. Beide Stoffströme wurden jeweils bei 500 mbar in einem Wärmetauscher verdampft und auf 350°C erhitzt. Zur besseren Aminverdampfung wurden 60 1/h Stickstoff als Trägergasstrom durch den Aminverdampfer geleitet. Hinter dem Reaktor wurde die Reaktionsmischung in siedendem o-Dichlorbenzol gequencht und mittels Stickstoffstrippung entphosgeniert. Die nach dem Versuch bestimmte Feststoffbildung am Amineintritt und im Reaktionsrohr belief sich auf 8,3 g.

Aufarbeitung wie unter Beispiel 6 beschrieben lieferte lediglich 16,6 g eines dunkelbraunen, hochviskosen Destillationsrückstandes, 49,8 g ISODI und 199,9 g OBDI als farbhelle Flüssigkeiten, die nach Ausweis kombinierter analytischer Methoden (NMR, GC-MS) zu >99% aus ISODI bzw. OBDI bestanden (68% bzw. 84% Ausbeute bezogen auf die eingesetzten Amine). Eine Verfärbung wurde bei beiden erfindungsgemäß erhaltenen Produkten auch nach wochenlanger Lagerung bei Zimmertemperatur unter Luftabschluß nicht beobachtet.

Mehrere Versuche, sowohl TEFUDA, IDDA als auch ISODA ohne jedwedes "Amin 2" mit Hilfe der GPP umzusetzen scheiterten nach maximal sechzig minütiger Laufzeit. Es bildeten sich hartnäckige Anbackungen und der Druckanstieg in der Anlage machte ein Abfahren nach unakzeptabel kurzer Laufzeit nötig. Das dabei in sehr schlechter Ausbeute gebildete ISODI war qualitativ völlig unakzeptabel und nur extrem aufwändig zu reinigen.

## Patentansprüche

1. Verfahren zur Herstellung von Isocyanaten durch Phosgenierung der korrespondierenden Amine, wobei ein Amin-Gemisch aus mindestens einem ersten Amin und mindestens einem von dem ersten Amin verschiedenen zweiten Amin mit Phosgen zu einem Reaktionsgemisch umgesetzt wird, **dadurch gekennzeichnet, dass**
entweder
• das erste Amin mindestens eine Ether-Gruppe enthält; und
• das zweite Amin ausgewählt ist aus der Gruppe, umfassend oder bestehend aus Aminoalkanen mit 5 bis 15 Kohlenwasserstoffatomen, welche keine Ether-Gruppen enthalten;
oder
• das erste Amin mindestens eine cyclische Ether-Gruppe enthält; und
• das zweite Amin mindestens eine offenkettige Ether-Gruppe und keine cyclischen Ether-Gruppen enthält.

2. Verfahren gemäß Anspruch 1, **dadurch gekennzeichnet, dass** das mindestens eine Ether-Gruppe enthaltende erste Amine eine Struktur der allgemeinen Formel X-(-R¹-NHₘ)ₙ aufweist, wobei
- X für H, NHₘ oder C(R²)ₚ;
- R¹ für, gegebenenfalls substituierte und/oder Heteroatom-aufweisende, aliphatische, (cyclo)aliphatische oder aromatische Reste mit vorzugsweise bis zu 10 Kohlenstoffatomen, wobei R¹ mindestens eine Ethergruppe enthält;
- R² für H, gegebenenfalls substituierte und/oder Heteroatom-aufweisende, aliphatische, (cyclo)aliphatische oder aromatische Reste mit vorzugsweise bis zu 10 Kohlenstoffatomen;
- m für 1 oder 2;
- n für 1, 2 oder 3; und
- p für 1, 2 oder 3
steht.

3. Verfahren gemäß Anspruch 1 oder 2, **dadurch gekennzeichnet, dass** das mindestens eine Ether-Gruppe enthaltende erste Amin, das mindestens eine cyclische Ether-Gruppe enthaltende erste Amin, das Aminoalkane mit 5 bis 15 Kohlenwasserstoffatome umfassende oder daraus bestehende zweite Amin und/oder das mindestens eine offenkettige Ether-Gruppe und keine cyclischen Ether-Gruppen enthaltene zweite Amin ein Diamin ist.

4. Verfahren gemäß einem der vorstehenden Ansprüche, **dadurch gekennzeichnet, dass** das mindestens eine Ether-Gruppe enthaltende erste Amin ausgewählt ist aus der Gruppe, umfassend oder bestehend aus (Tetrahydrofuran-2,5-diyl)dimethanamin, 3,6-diamino-hexahydrofuro[3,2-b]furan, 3,6-Bis(aminomethyl)-hexahydrofuro[3,2-b]furan, Furan-2,5-diyldimethanamin, (Methan-2,2-diylbis(tetrahydrofuran-5,2-diyl))dimethanamin, (Methan-2,2-diylbis(furan-5,2-diyl))dimethanamin, (Propan-2,2-diylbis(tetrahydrofuran-5,2-diyl))dimethanamin, (Propan-2,2-diylbis(furan-5,2-diyl))dimethanamin; Di(aminoethyl)ether, insbesondere 2,2'-oxybis(ethan-1-amin), 1,1'-oxybis(propan-2-amin), 2-(2-aminoethoxy)propan-1-amin, 2,2'-oxybis(propan-1-amin) und 2-(2-aminopropoxy)propan-1-amin; Di(aminopropyl)ether oder Mischungen davon und/oder
das mindestens eine cyclische Ether-Gruppe enthaltende erste Amin ausgewählt ist aus der Gruppe, umfassend oder bestehend aus (Tetrahydrofuran-2,5-diyl)dimethanamin, 3,6-diamino-hexahydrofuro[3,2-b]furan, 3,6-Bis(aminomethyl)-hexahydrofuro[3,2-b]furan, Furan-2,5-diyldimethanamin, (Methan-2,2-diylbis(tetrahydrofuran-5,2-diyl))dimethanamin, (Methan-2,2-diylbis(furan-5,2-diyl))dimethanamin, (Propan-2,2-diylbis(tetrahydrofuran-5,2-diyl))dimethanamin, (Propan-2,2-diylbis(furan-5,2-diyl))dimethanamin oder Mischungen davon.

5. Verfahren gemäß einem der vorstehenden Ansprüche, **dadurch gekennzeichnet, dass** das Aminoalkan mit 5 bis 15 Kohlenwasserstoffatomen umfassende oder daraus bestehende zweite Amin ausgewählt ist aus der Gruppe, umfassend oder bestehend aus 1,5-Diaminopentan; 1,6-Diaminohexan; 1-Amino-3,5,5-trimethyl-5-aminomethylcyclohexan; Amino-[(aminocyclohexyl)methyl]cyclohexan, insbesondere 4,4'-Methylenbis(cyclohexan-1-amin), 2-((4-Aminocyclohexyl)methyl)cyclohexan-1-amin und 2,2'-Methylenbis(cyclohexan-1-amin); 1,3-Bis(aminomethyl)cyclohexan, 1,4-Bis(aminomethyl)cyclohexan; Diaminocyclohexan, insbesondere Cyclohexan-1,2-diamin, Cyclohexan-1,3-diamin und Cyclohexan-1,4-diamin; Methyl-diamino-cyclohexan, insbesondere 4-Methylcyclohexan-1,3-diamin und 2-Methylcyclohexan-1,3-diamin, 4,4'-methylenbis(2-methylcyclohexan-1-amin); oder Mischungen davon.

6. Verfahren gemäß einem der vorstehenden Ansprüche, **dadurch gekennzeichnet, dass** das mindestens eine offenkettige Ether-Gruppen und keine cyclischen Ether-Gruppen enthaltende zweite Amin ausgewählt ist aus der Gruppe, umfassend oder bestehend aus Di(aminoethyl)ether, Di(aminopropyl)ether, 1,8-Diamino-1,5,8-trimethyl-3,6-dioxaoctan, 1,11-Diamino-1,5,8,11-tetramethylundecan, 1,8-Diamino-3,6-dioxaoktan, 1,10-Diamino-4,7-dioxadecan, 1,12-Diamino-4,9-dioxadodecan, 1,14-Diamino-3,10-dioxatetradecan, 1,13-Diamino-4,7,10-trioxatridecanan, 1,7-Diamino-2,6-dioxa-4-aminomethoxy-heptan, 1-Amino-2-oxa-3,3-bis(aminomethoxy)hexan, 1,9-Diamino-3,7-dioxa-5-(1-amino-2-ethoxy)-nonan, 1-Amino-3-oxa-4,4-bis(1-amino-2-ethoxy)heptan, 1,11-Diamino-4,8-dioxa-6-(1-amino-5-oxabutyl)undecan, 1-Amino-4-oxa-5,5-bis(1-amino-5-oxabutyl)oktan oder Mischungen davon.

7. Verfahren gemäß einem der vorstehenden Ansprüche, **dadurch gekennzeichnet, dass** der Massenanteil
• des ersten Amins 5,0 bis 95,0 Gew.-%, bevorzugt 10,0 bis 80,0 Gew.-% und
• des zweiten Amins 95,0 bis 5,0 Gew.-%, bevorzugt 90,0 bis 20,0 Gew.-%, weiter bevorzugt 80,0 bis 20,0
beträgt, bezogen auf das Gesamtgewicht des Amin-Gemischs.

8. Verfahren gemäß einem der vorstehenden Ansprüche, **dadurch gekennzeichnet, dass** das molare Verhältnis von Phosgen zu den Aminogruppen der Amine des Amin-Gemischs ≥ 1:1 bis ≤ 5:1, bevorzugt > 1:1 bis ≤ 3:1 beträgt.

9. Verfahren gemäß einem der vorstehenden Ansprüche, **dadurch gekennzeichnet, dass** bei der Umsetzung ein Inertstoff eingesetzt wird, wobei der Inertstoff ausgewählt ist aus der Gruppe, umfassend oder bestehend aus
• Intergasen, insbesondere Stickstoff, Argon oder Mischung von diesen;
• inerten Lösungsmitteln, insbesondere aromatische Kohlenwasserstoffe, bevorzugt Chlorbenzol, o-Dichlorbenzol, Toluol, Xylol oder Mischungen von diesen;
• oder Mischungen von den vorgenannten Inertgasen und inerten Lösungsmitteln.

10. Verfahren gemäß einem der vorstehenden Ansprüche, **dadurch gekennzeichnet, dass** das zum ersten Amin korrespondierende Isocyanat und/oder das zum zweiten Amin korrespondierende Isocyanat, von dem Reaktionsgemisch abgetrennt wird, wobei die Abtrennung vorzugsweise mittels Destillation, insbesondere Dünnschichtdestillation, Extraktion, Kristallisation, Umkristallisation oder einer Kombination von diesen erfolgt und die jeweiligen Isocyanate getrennt oder als Isocyanat-Gemisch erhalten werden.

11. Verfahren gemäß Anspruch 10, **dadurch gekennzeichnet, dass** der Gehalt an aciden Chlorverbindungen in dem/den abgetrennten Isocyanat(en) oder dem Isocyanat-Gemisch von 1 bis 100 ppm, bevorzugt von 2 bis 80 ppm, besonders bevorzugt von 5 bis 50 ppm beträgt, bestimmt gemäß der Norm ISO 15028:2014, und/oder der Gehalt an hydrolysierbarem Chlor in dem/den abgetrennten Isocyanat(en) oder dem Isocyanat-Gemisch von 1 bis 500 ppm, bevorzugt von 5 bis 100 ppm beträgt, bestimmt gemäß der Norm ISO 15028:2014.

12. Verwendung eines Amin-Gemischs aus mindestens einem ersten Amin und mindestens einem von dem ersten Amin unterschiedlichen zweiten Amin in einem Verfahren zur Herstellung der korrespondierenden Isocyanate durch Phosgenierung nach einem Verfahren gemäß einem der Ansprüche 1 bis 11 zur Erniedrigung des Gehaltes an aciden Chlorverbindungen und/oder des Gehaltes an hydrolysierbarem Chlor der bei der Herstellung erhaltenen korrespondierenden Isocyanate.

13. Produkt erhältlich oder erhalten durch ein Verfahren gemäß einem der Ansprüche 1 bis 11, vorzugsweise unmittelbar erhältlich oder erhalten durch ein Verfahren gemäß einem der Ansprüche 1 bis 11.

14. Verwendung des Produkts gemäß Anspruch 13 und/oder des Isocyanats oder Isocyanat-Gemischs erhalten oder erhältlich nach einem Verfahren gemäß Anspruch 10 oder 11 als Komponente zur Herstellung von Polyurethanen, insbesondere Polyurethan-Schaumstoffen, Polyurethan-Beschichtungen und Polyurethan-Klebstoffen, von pharmazeutischen Erzeugnissen, insbesondere Wirkstoffen, sowie von Hilfsstoffen, insbesondere von Hilfsstoffen für die Nassfestausrüstung von Papier und anderen Zelluloseprodukten, Emulgatoren und Verdickungsmitteln.

15. Phosgenierungsanlage zur Durchführung des Verfahrens gemäß den Ansprüchen 1 bis 11, umfassend oder bestehend aus
(a) mindestens eine Phosgen-Vorrichtung zur kontinuierlichen Bereitstellung von Phosgen, gegebenenfalls in Kombination mit einem Inertstoff
(b) gegebenenfalls eine Inertstoff-Vorrichtung zur Bereitstellung eines Inertstoffs,
(c) mindestens eine Amin-Vorrichtung zur kontinuierlichen Bereitstellung eines Amin-Gemischs, gegebenenfalls in Kombination mit einem Inertstoff
(d) einen Phosgenierungsreaktor zur Vermischung des Phosgens und des Amin-Gemischs sowie gegebenenfalls des Inertstoffs und Umsetzung des Amin-Gemischs mit dem Phosgen,
**dadurch gekennzeichnet, dass** die Amin-Vorrichtung einen ersten Vorlagebehälter für ein erstes Amin und einen zweiten Vorlagebehälter für ein zweites Amin sowie eine Dosiereinrichtung zur unabhängig voneinander variablen Einstellung der Massenanteile des ersten Amins und des zweiten Amins und optional eine Mischeinrichtung zur Vermischung des ersten Amins und des zweiten Amins zu dem Amin-Gemisch umfasst.
